# EUROPEAN PATENT SPECIFICATION

(11) **EP 3 481 505 B1**
(45) Date of publication and mention of the grant of the patent: **03.09.2025**
(21) Application number: 17825031.2
(22) Date of filing: 07.07.2017
(51) Int. Cl.: A61K 31/135, A61K 31/137, A61K 31/197, A61K 31/198, A61K 31/44, A61K 31/4415, A61P 9/06

(54) **PREVENTION AND TREATMENT OF ATRIAL FIBRILLATION/FLUTTER WITH GAMMA-KETOALDEHYDE SCAVENGERS**
VORBEUGUNG UND BEHANDLUNG VON VORHOFFLIMMERN/FLATTERN MIT GAMMA-KETOALDEHYD-FÄNGERN
PRÉVENTION ET TRAITEMENT DE LA FIBRILLATION/FLUTTER AURICULAIRE AVEC DES CAPTEURS DE GAMMA-CÉTOALDÉHYDE

(30) Priority: 07.07.2016 US 201662359705 P
(43) Date of publication of application: 15.05.2019
(73) Proprietor: Vanderbilt University, Nashville, TN 37240 (US)
(72) Inventor: MURRAY, Katherine T., Brentwood, Tennessee 37027 (US); ROBERTS, L. Jackson II, Gallatin, Tennessee 37066 (US); AMARNATH, Venkataraman, Brentwood, Tennessee 37027 (US)
(74) Representative: Mewburn Ellis LLP
(86) International application number: PCT/US2017/041211
(87) International publication number: WO 2018/009875

(56) References cited:
- WO-A1-2011/008202
- US-A1- 2007 249 562
- US-A1- 2012 157 501
- US-B1- 6 620 836
- T.N. SIDOROVA, ET AL.: "Reactive [gamma]-ketoaldehydes promote protein misfolding and preamyloid oligomer formation in rapidly-activated atrial cells", JOURNAL OF MOLECULAR AND CELLULAR CARDIOLOGY, vol. 79, 18 November 2014 (2014-11-18), Elsevier, Oxford, GB, pages 295 - 302, XP055657947, ISSN: 0022-2828, DOI: 10.1016/j.yjmcc.2014.11.013

## Description

### BACKGROUND AND SUMMARY OF THE INVENTION

Atrial fibrillation (AF) is the most common cardiac arrhythmia of clinical significance, and it often results in devastating outcomes. Because current treatment is frequently ineffective, there is a critical need for an improved understanding of the molecular mechanisms causing AF and novel strategies to treat it.
Methods of treating atrial fibrillation in a mammal that includes administering a therapeutically effective amount of pyridoxal-5'-phosphate, pyridoxic acid, pyridoxamine, pyridoxal or 3-acylated pyridoxal analogues are described in US 2007/0249562 A1.

However, the present inventors have linked inflammation and oxidative stress to the pathogenesis and progression of AF. Unfortunately, antioxidants as "upstream therapy" (including vitamins C and E, statins, and inhibitors of the renin-angiotensin-aldosterone system) have been ineffective in clinical trials, highlighting a limited understanding of the appropriate molecular targets and/or treatment strategies. Recently, highly-reactive mediators of oxidative stress have been identified that participate in oxidative injury that occurs in the cardiovascular system and brain. **In** the presence of oxidative injury, arachidonic acid can undergo oxygenation and structural rearrangement to generate γ-ketoaldehydes (y-KAs), also described as isolevuglandins (IsoLGs). These compounds are the most reactive products of lipid peroxidation identified to date, and they rapidly adduct to lysine residues of proteins to form stable adducts and intermolecular crosslinks. γ-KA adducts are increased in multiple pathologic conditions, including Alzheimer's disease and hypertension, linked to oxidative injury and inflammation. Unlike reactive oxygen species (ROS) that participate in physiologic processes such as cell signaling, there are no physiologic/beneficial effects that have been attributed to γ-KAs. Rather, they have been shown to directly promote the aggregation of amyloid β₁₋₄₂ (linked to Alzheimer's) into cytotoxic protein The compounds for use according to the present invention rapidly bind γ-KAs to "scavenge" these injurious mediators to prevent oxidative protein modification, as an alternative approach to upstream therapy. One of the compounds, for use, according to the present invention, salicylamine, is a natural product with an excellent safety profile in pre-clinical animal studies. Moreover, salicylamine prevents the formation of both γ-KAs and toxic protein oligomers with remarkable therapeutic benefit in animal models of Alzheimer's disease and hypertension. The present inventors have identified protein oligomers and oxidative stress/formation of γ-KAs in cellular and *in vivo* models associated with AF susceptibility, including rapidly-stimulated atrial cells, hypertension, obesity, and familial AF. Importantly, our preliminary data demonstrate a beneficial effect of scavenging γ-KAs to reduce atrial protein oligomer formation and AF burden. Therefore, salicylamine, as well as its structural analogues that have been developed to date, represent a completely novel therapy to prevent and treat atrial arrhythmias, such as atrial fibrillation/atrial flutter.

### DESCRIPTION OF THE FIGURES

Figure 1 is a flow chart that links oxidative stress with IsoLG/PAO formation, and ultimately atrial arrhythmogenesis.
Figure 2 is shows the chemical mechanism for the scavenging of the1,4-dicarbonyl (red box) compounds known as isolevuglandins by pyridoxamine and its structural analog 2-HOBA (center box).
Figure 3 shows colocalization of ANP and PAO immunoreactivity. Immunolabeling with PAO-(A-11; A) and ANP-specific (B) antibodies in adjacent 5µm human atrial samples. C. Binary mask, or area of myocardium. D. PAO (green) and ANP (red) signals within myocardium in adjacent sections. Scale bars=50µm.
Figure 4 shows IsoLGs in PAO formation for atrial HL-1 cells. After incubation with synthetic IsoLGs (isoketals) for 6hr, PAO formation was evident in unpaced cells (top panels). The anti-IsoLG adduct antibody (D11 ScFv) is immunoreactive in paced but not control cells (bottom panels).
Figure 5 shows atrial IsoLG adducts (A and B [preliminary mass spectrometry data; n=1 each, similar results with 1 or 3 pooled whole atria]) and PAOs (C and D) formed during angII-mediated HTN. Scale bar=50µm.
Figure 6 shows ANPoligomers partially co-localize with atrial PAOs and are cytoxic to atrial cells. (A-D) Format as in Figure 3. Scale bars = 50µm. (E) Western blot of ANP after peptide incubation (10µM) for 24hr or 6d, compared to incubation with IsoLGs for 24hr. (F) ANP oligomers (2wk incubation) reduced ATP production by atrial HL-1 cells (24hr exposure).
Figure 7 demonstrates that hypertension-mediated AF is suppressed by the dicarbonyl scavenger 2-HOBA. (A) Total inducible AF burden for normotensive (sham) and hypertensive (ang II) mice, as well as hypertensive mice treated with 2-HOBA, 4-HOBA or hydralazine/ hydrocholorothiazide (hyd/HCTZ; n=13, 17, 14, 7, and 7 respectively; ^{*}P<0.05, ^{**}P<0.01). (B) Blood pressure data are shown for each experimental group over time.
Figure 8 demonstrates that 2-HOBA prevents formation of IsoLG adducts and PAOs during angII-mediated hypertension. (A) Representative images for PAOs (A11 or top row), myocardium (MF20 or middle row), and IsoLG adducts (D-11; bottom row) in normotensive (sham) and hypertensive (angII) mice, and hypertensive mice treated with 2-HOBA. Scale bars = 50µm. (B) Summary data for PAO burden (expressed as G/R values).
Figure 9 shows IsoLG adducts quantified in atrial HL-1 cells under cyclical 10% stretch (24hr) and unstretched conditions.
Figure 10 demonstrates that DIO-mediated AF is suppressed by 2-HOBA. (A) Total inducible AF burden is shown in mice fed a fow-fat (LFD) or high-fat (HFD) diet, as well as HFD mice treated with 2-HOBA or 4-HOBA (n=10 each; ^{*}P<0.05, ^{**}P<0.01). (B) Weight data are illustrated for each experimental group over time.
Figure 11 shows that obesity causes atrial PAOs. A, B. Images for merged and final PAO signal in LA of mice fed a low-fat (LFD) or high-fat diet (HFD; 8wk).
Figure 12 shows PAO formation in atria of NTG, *NPPA* (WT), and m-NPPA (mutant) mice. Immunolabeling with PAO and myocardium-specific antibodies (lower panel=summary data).
Figure 13 shows enhanced fibrillogenesis of mutant ANP peptide compared to wild-type ANP. ANP Western blot of wild-type (WT) and mutant (Mut) ANP after incubation (10µM) for 24hr in the absence/presence of IsoLGs, 6d, and 10d (single blot, gaps=empty lane removal).
Figure 14 shows Atrial HL-1 cell ATP production with WT and m-ANP incubated for variable time periods.
Figure 15 is an example demonstrating a diet-induced obesity model.

### Description of the Invention

Before the present compounds, compositions, articles, systems, devices, and/or methods are disclosed and described, it is to be understood that they are not limited to specific synthetic methods unless otherwise specified, or to particular reagents unless otherwise specified, as such may, of course, vary. It is also to be understood that the terminology used herein is for the purpose of describing particular aspects only and is not intended to be limiting. Although any methods and materials similar or equivalent to those described herein can be used in the practice or testing of the present invention, example methods and materials are now described.
The subject matter of the present invention is defined in the claims.
The publications discussed herein are provided solely for their disclosure prior to the filing date of the present application. Nothing herein is to be construed as an admission that the present invention is not entitled to antedate such publication by virtue of prior invention. Further, the dates of publication provided herein can be different from the actual publication dates, which need to be independently confirmed.

As used in the specification and the appended claims, the singular forms "a," "an" and "the" include plural referents unless the context clearly dictates otherwise. Thus, for example, reference to "a functional group," "an alkyl," or "a residue" includes mixtures of two or more such functional groups, alkyls, or residues, and the like.

Ranges can be expressed herein as from "about" one particular value, and/or to "about" another particular value. When such a range is expressed, a further aspect includes from the one particular value and/or to the other particular value. Similarly, when values are expressed as approximations, by use of the antecedent "about," it will be understood that the particular value forms a further aspect. It will be further understood that the endpoints of each of the ranges are significant both in relation to the other endpoint, and independently of the other endpoint. It is also understood that there are a number of values disclosed herein, and that each value is also herein disclosed as "about" that particular value in addition to the value itself. For example, if the value "10" is disclosed, then "about 10" is also disclosed. It is also understood that each unit between two particular units are also disclosed. For example, if 10 and 15 are disclosed, then 11, 12, 13, and 14 are also disclosed.

As used herein, the terms "optional" or "optionally" means that the subsequently described event or circumstance can or can not occur, and that the description includes instances where said event or circumstance occurs and instances where it does not.

As used herein, the term "subject" refers to a target of administration. The subject of the herein disclosed methods can be a vertebrate, such as a mammal, a fish, a bird, a reptile, or an amphibian. Thus, the subject of the herein disclosed methods can be a human, non-human primate, horse, pig, rabbit, dog, sheep, goat, cow, cat, guinea pig or rodent. The term does not denote a particular age or sex. Thus, adult and newborn subjects, as well as fetuses, whether male or female, are intended to be covered. A patient refers to a subject afflicted with a disease or disorder. The term "patient" includes human and veterinary subjects.

As used herein, the term "treatment" refers to the medical management of a patient with the intent to cure, ameliorate, stabilize, or prevent a disease, pathological condition, or disorder. This term includes active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder.

As used herein, the term "prevent" or "preventing" refers to precluding, averting, obviating, forestalling, stopping, or hindering something from happening, especially by advance action. It is understood that where reduce, inhibit or prevent are used herein, unless specifically indicated otherwise, the use of the other two words is also expressly disclosed. As can be seen herein, there is overlap in the definition of treating and preventing.

As used herein, the term "diagnosed" means having been subjected to a physical examination by a person of skill, for example, a physician, and found to have a condition that can be diagnosed or treated by the compounds, compositions, or methods disclosed herein. As used herein, the phrase "identified to be in need of treatment for a disorder," or the like, refers to selection of a subject based upon need for treatment of the disorder. For example, a subject can be identified as having a need for treatment of a disorder (e.g., a disorder related to inflammation) based upon an earlier diagnosis by a person of skill and thereafter subjected to treatment for the disorder. It is contemplated that the identification can, in one aspect, be performed by a person different from the person making the diagnosis. It is also contemplated, in a further aspect, that the administration can be performed by one who subsequently performed the administration.

As used herein, the terms "administering" and "administration" refer to any method of providing a pharmaceutical preparation to a subject. Such methods are well known to those skilled in the art and include, but are not limited to, oral administration, transdermal administration, administration by inhalation, nasal administration, topical administration, intravaginal administration, ophthalmic administration, intraaural administration, intracerebral administration, rectal administration, and parenteral administration, including injectable such as intravenous administration, intra-arterial administration, intramuscular administration, and subcutaneous administration. Administration can be continuous or intermittent. In various aspects, a preparation can be administered therapeutically; that is, administered to treat an existing disease or condition. In further various aspects, a preparation can be administered prophylactically; that is, administered for prevention of a disease or condition.

As used herein, the term "effective amount" refers to an amount that is sufficient to achieve the desired result or to have an effect on an undesired condition. For example, a "therapeutically effective amount" refers to an amount that is sufficient to achieve the desired therapeutic result or to have an effect on undesired symptoms, but is generally insufficient to cause adverse side effects. The specific therapeutically effective dose level for any particular patient will depend upon a variety of factors including the disorder being treated and the severity of the disorder; the specific composition employed; the age, body weight, general health, sex and diet of the patient; the time of administration; the route of administration; the rate of excretion of the specific compound employed; the duration of the treatment; drugs used in combination or coincidental with the specific compound employed and like factors well known in the medical arts. For example, it is well within the skill of the art to start doses of a compound at levels lower than those required to achieve the desired therapeutic effect and to gradually increase the dosage until the desired effect is achieved. If desired, the effective daily dose can be divided into multiple doses for purposes of administration. Consequently, single dose compositions can contain such amounts or submultiples thereof to make up the daily dose. The dosage can be adjusted by the individual physician in the event of any contraindications. Dosage can vary, and can be administered in one or more dose administrations daily, for one or several days. Guidance can be found in the literature for appropriate dosages for given classes of pharmaceutical products. In further various aspects, a preparation can be administered in a "prophylactically effective amount"; that is, an amount effective for prevention of a disease or condition.

As used herein, the term "pharmaceutically acceptable carrier" refers to sterile aqueous or nonaqueous solutions, dispersions, suspensions or emulsions, as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol and the like), carboxymethylcellulose and suitable mixtures thereof, vegetable oils (such as olive oil) and injectable organic esters such as ethyl oleate. Proper fluidity can be maintained, for example, by the use of coating materials such as lecithin, by the maintenance of the required particle size in the case of dispersions and by the use of surfactants. These compositions can also contain adjuvants such as preservatives, wetting agents, emulsifying agents and dispersing agents. Prevention of the action of microorganisms can be ensured by the inclusion of various antibacterial and antifungal agents such as paraben, chlorobutanol, phenol, sorbic acid and the like. It can also be desirable to include isotonic agents such as sugars, sodium chloride and the like. Prolonged absorption of the injectable pharmaceutical form can be brought about by the inclusion of agents, such as aluminum monostearate and gelatin, which delay absorption. Injectable depot forms are made by forming microencapsule matrices of the drug in biodegradable polymers such as polylactide-polyglycolide, poly(orthoesters) and poly(anhydrides). Depending upon the ratio of drug to polymer and the nature of the particular polymer employed, the rate of drug release can be controlled. Depot injectable formulations are also prepared by entrapping the drug in liposomes or microemulsions which are compatible with body tissues. The injectable formulations can be sterilized, for example, by filtration through a bacterial-retaining filter or by incorporating sterilizing agents in the form of sterile solid compositions which can be dissolved or dispersed in sterile water or other sterile injectable media just prior to use. Suitable inert carriers can include sugars such as lactose. Desirably, at least 95% by weight of the particles of the active ingredient have an effective particle size in the range of 0.01 to 10 micrometers.

As used herein, the term "scavenger" or "scavenging" refers to a chemical substance that can be administered in order to remove or inactivate impurities or unwanted reaction products. For example, the isoketals irreversibly adduct specifically to lysine residues on proteins. The isoketal scavengers of the present invention react with isoketals before they adduct to the lysine residues. Accordingly, the compounds of the present invention "scavenge" isoketals, thereby preventing them from adducting to proteins.

As used herein, the term "substituted" is contemplated to include all permissible substituents of organic compounds. In a broad aspect, the permissible substituents include acyclic and cyclic, branched and unbranched, carbocyclic and heterocyclic, and aromatic and nonaromatic substituents of organic compounds. Illustrative substituents include, for example, those described below. The permissible substituents can be one or more and the same or different for appropriate organic compounds. For purposes of this disclosure, the heteroatoms, such as nitrogen, can have hydrogen substituents and/or any permissible substituents of organic compounds described herein which satisfy the valences of the heteroatoms. This disclosure is not intended to be limited in any manner by the permissible substituents of organic compounds. Also, the terms "substitution" or "substituted with" include the implicit proviso that such substitution is in accordance with permitted valence of the substituted atom and the substituent, and that the substitution results in a stable compound, *e.g*., a compound that does not spontaneously undergo transformation such as by rearrangement, cyclization, elimination, etc.

The term "alkyl" as used herein is a branched or unbranched saturated hydrocarbon group of 1 to 24 carbon atoms, such as methyl, ethyl, n-propyl, isopropyl, n-butyl, isobutyl, s-butyl, t-butyl, n-pentyl, isopentyl, s-pentyl, neopentyl, hexyl, heptyl, octyl, nonyl, decyl, dodecyl, tetradecyl, hexadecyl, eicosyl, tetracosyl, and the like. The alkyl group can be cyclic or acyclic. The alkyl group can be branched or unbranched. The alkyl group can also be substituted or unsubstituted. For example, the alkyl group can be substituted with one or more groups including, but not limited to, optionally substituted alkyl, cycloalkyl, alkoxy, amino, ether, halide, hydroxy, nitro, silyl, sulfo-oxo, or thiol, as described herein. A "lower alkyl" group is an alkyl group containing from one to six (e.g., from one to four) carbon atoms.

Throughout the specification "alkyl" is generally used to refer to both unsubstituted alkyl groups and substituted alkyl groups; however, substituted alkyl groups are also specifically referred to herein by identifying the specific substituent(s) on the alkyl group. For example, the term "halogenated alkyl" specifically refers to an alkyl group that is substituted with one or more halide, *e.g*., fluorine, chlorine, bromine, or iodine. The term "alkoxyalkyl" specifically refers to an alkyl group that is substituted with one or more alkoxy groups, as described below. The term "alkylamino" specifically refers to an alkyl group that is substituted with one or more amino groups, as described below, and the like. When "alkyl" is used in one instance and a specific term such as "alkylalcohol" is used in another, it is not meant to imply that the term "alkyl" does not also refer to specific terms such as "alkylalcohol" and the like.

This practice is also used for other groups described herein. That is, while a term such as "cycloalkyl" refers to both unsubstituted and substituted cycloalkyl moieties, the substituted moieties can, in addition, be specifically identified herein; for example, a particular substituted cycloalkyl can be referred to as, *e.g.,* an "alkylcycloalkyl." Similarly, a substituted alkoxy can be specifically referred to as, *e.g.,* a "halogenated alkoxy," a particular substituted alkenyl can be, *e.g.,* an "alkenylalcohol," and the like. Again, the practice of using a general term, such as "cycloalkyl," and a specific term, such as "alkylcycloalkyl," is not meant to imply that the general term does not also include the specific term.

The term "cycloalkyl" as used herein is a non-aromatic carbon-based ring composed of at least three carbon atoms. Examples of cycloalkyl groups include, but are not limited to, cyclopropyl, cyclobutyl, cyclopentyl, cyclohexyl, norbornyl, and the like. The term "heterocycloalkyl" is a type of cycloalkyl group as defined above, and is included within the meaning of the term "cycloalkyl," where at least one of the carbon atoms of the ring is replaced with a heteroatom such as, but not limited to, nitrogen, oxygen, sulfur, or phosphorus. The cycloalkyl group and heterocycloalkyl group can be substituted or unsubstituted. The cycloalkyl group and heterocycloalkyl group can be substituted with one or more groups including, but not limited to, optionally substituted alkyl, cycloalkyl, alkoxy, amino, ether, halide, hydroxy, nitro, silyl, sulfo-oxo, or thiol as described herein.

The term "polyalkylene group" as used herein is a group having two or more CH₂ groups linked to one another. The polyalkylene group can be represented by a formula -(CH₂)ₐ-, where "a" is an integer of from 2 to 500.

The terms "alkoxy" and "alkoxyl" as used herein to refer to an alkyl or cycloalkyl group bonded through an ether linkage; that is, an "alkoxy" group can be defined as -OA¹ where A¹ is alkyl or cycloalkyl as defined above. "Alkoxy" also includes polymers of alkoxy groups as just described; that is, an alkoxy can be a polyether such as -OA¹-OA² or -OA¹-(OA²)ₐ-OA³, where "a" is an integer of from 1 to 200 and A¹, A², and A³ are alkyl and/or cycloalkyl groups.

The terms "amine" or "amino" as used herein are represented by a formula NA¹A²A³, where A¹, A², and A³ can be, independently, hydrogen or optionally substituted alkyl, cycloalkyl, alkenyl, cycloalkenyl, alkynyl, cycloalkynyl, aryl, or heteroaryl group as described herein.

The term "hydroxyl" as used herein is represented by a formula -OH.

The term "nitro" as used herein is represented by a formula -NO₂.

The term "pharmaceutically acceptable" describes a material that is not biologically or otherwise undesirable, i.e., without causing an unacceptable level of undesirable biological effects or interacting in a deleterious manner.

Embodiments of the present invention include a pharmaceutical composition comprising a pharmaceutically acceptable carrier and a compound of the following formula, for use in the treatment, prevention, or amelioration of:
i) atrial fibrillation in a subject with or at risk of atrial fibrillation, thereby inhibiting or treating the atrial fibrillation; or
ii) atrial arrhythmias in a subject with or at risk of an atrial arrhythmia 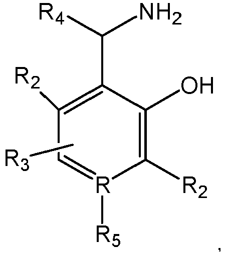 wherein:
   R is C;
   R₂ is independently H, hydroxy, halogen, nitro, CF₃, C₁₋₆ alkyl, C₁₋₆ alkoxy, C₃₋₁₀ cycloalkyl, C₃₋₈ membered ring containing C, O, S or N, optionally substituted with one or more R₂, R₃ and R_{4,} and may cyclize with one or more R_{2,} R₃, or R₅ to form an optionally substituted C₃₋₈ membered ring containing C, O, S or N;
   R₃ is H, hydroxy, halogen, nitro, CF₃, C₁₋₆ alkyl, C₁₋₆ alkoxy, C₃₋₁₀ cycloalkyl, C₃₋₈ membered ring containing C, O, S or N, optionally substituted with one or more R₄, R₂ and R₃ may cyclize with one or more R₂ or R₅ to form an optionally substituted C₃₋₈ membered ring containing C, O, S or N;
   R₄ is H, hydroxy, halogen, nitro, CF₃, C₁₋₆ alkyl, C₁₋₆ alkoxy, C₃₋₁₀ cycloalkyl, C₃₋₈ membered ring containing C, O, S or N, optionally substituted with one or more R₄, R₂ and R₃ may cyclize with one or more R_{2,} R_{3,} or R₅ to form an optionally substituted C₃₋₈ membered ring containing C, O, S or N;
   R₅ is, H, hydroxy, halogen, nitro, CF₃, C₁₋₆ alkyl, C₁₋₆ alkoxy, C₃₋₁₀ cycloalkyl, C₃₋₈ membered ring containing C, O, S or N, optionally substituted with one or more R₄, R₂ and R₃ may cyclize with one or more R_{2,} R_{3,} or R₄ to form an optionally substituted C₃₋₈ membered ring containing C, O, S or N;
   and stereoisomers thereof.

In certain embodiments, the compound for use may be selected from the compounds disclosed herein.

In a preferred embodiment, the compound for use may be salicylamine.

Another embodiment of the present invention is a compound of the following formula for use in treatment, prevention, or amelioration of atrial fibrillation in a subject with or at risk of atrial fibrillation, thereby inhibiting or treating the atrial fibrillation, the compound being co-administered to the subject with a drug having a known side effect of treating, preventing, or ameliorating atrial fibrillation 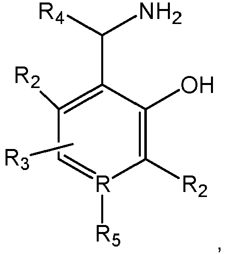 wherein:
R is C;
R₂ is independently H, hydroxy, halogen, nitro, CF₃, C₁₋₆ alkyl, C₁₋₆ alkoxy, C₃₋₁₀ cycloalkyl, C₃₋₈ membered ring containing C, O, S or N, optionally substituted with one or more R₂, R₃ and R_{4,} and may cyclize with one or more R_{2,} R₃, or R₅ to form an optionally substituted C₃₋₈ membered ring containing C, O, S or N;
R₃ is H, hydroxy, halogen, nitro, CF₃, C₁₋₆ alkyl, C₁₋₆ alkoxy, C₃₋₁₀ cycloalkyl, C₃₋₈ membered ring containing C, O, S or N, optionally substituted with one or more R₄, R₂ and R₃ may cyclize with one or more R₂ or R₅ to form an optionally substituted C₃₋₈ membered ring containing C, O, S or N;
R₄ is H, hydroxy, halogen, nitro, CF₃, C₁₋₆ alkyl, C₁₋₆ alkoxy, C₃₋₁₀ cycloalkyl, C₃₋₈ membered ring containing C, O, S or N, optionally substituted with one or more R₄, R₂ and R₃ may cyclize with one or more R_{2,} R_{3,} or R₅ to form an optionally substituted C₃₋₈ membered ring containing C, O, S or N;
R₅ is, H, hydroxy, halogen, nitro, CF₃, C₁₋₆ alkyl, C₁₋₆ alkoxy, C₃₋₁₀ cycloalkyl, C₃₋₈ membered ring containing C, O, S or N, optionally substituted with one or more R₄, R₂ and R₃ may cyclize with one or more R_{2,} R_{3,} or R₄ to form an optionally substituted C₃₋₈ membered ring containing C, O, S or N; and stereoisomers thereof.

Examples of compounds for use as disclosed herein may include but are not limited to, compounds selected from the formula: 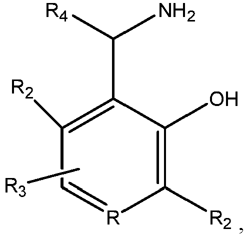 wherein:
R is CH;
R₂ is independently H, or substituted or unsubstituted C₁₋₆ alkyl;
R₃ is H, halogen, C₁₋₆ alkoxy, hydroxyl, or nitro;
R₄ is H, substituted or unsubstituted C₁₋₆ alkyl, or carboxyl; and pharmaceutically acceptable salts thereof.

In a preferred embodiment, the compound for use is salicylamine (2-hydroxybenzylamine or 2-HOBA). The compound for use may be chosen from: 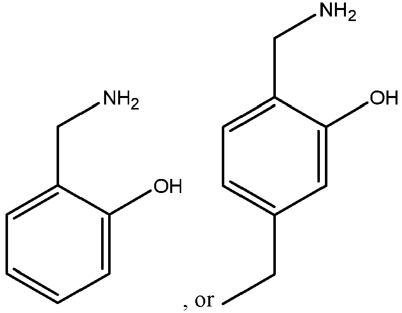 or a pharmaceutically acceptable salt thereof.

The compound for use may also be chosen from: 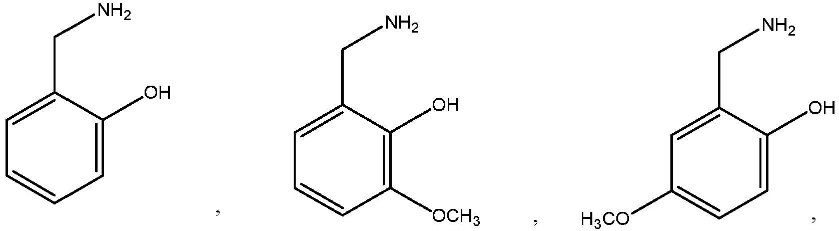 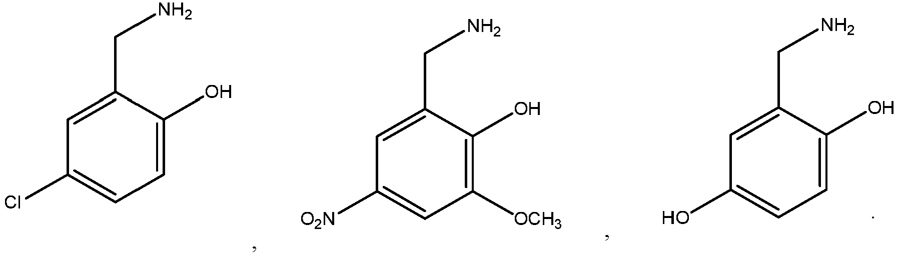 or a pharmaceutically acceptable salt thereof.

The compounds for use may also be chosen from: 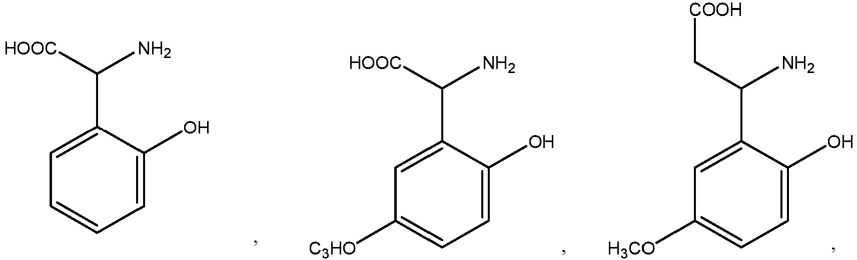 or a pharmaceutically acceptable salt thereof.

The compounds for use may also be chosen from 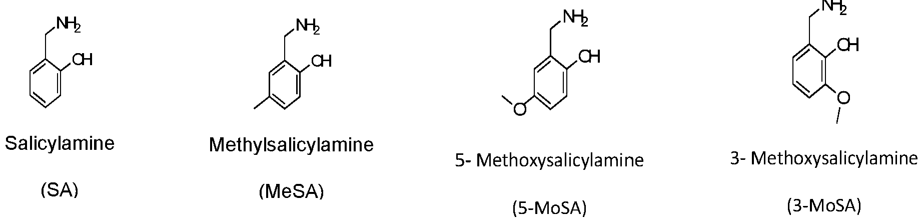 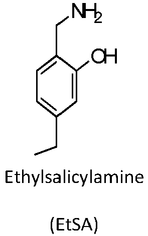 or a pharmaceutically acceptable salt thereof.

One in four persons age 40 years and older will develop atrial fibrillation (AF), a refractory arrhythmia that often results in devastating clinical outcomes. AF confers a 2-fold increased risk of dying, and substantial morbidity in the form of stroke, congestive heart failure, and treatment complications such as anticoagulant-related bleeding. The prevalence of AF continues to increase, highlighting its emergence as a growing epidemic and major public health challenge in the US and worldwide. The estimated annual cost of AF-related hospitalizations in 2010 was $3.46 billion dollars in the US alone.

Accordingly, there is a long felt need for the present invention.

A major barrier of contemporary AF treatment has been the focus to reduce arrhythmia recurrence, using antiarrhythmic drugs and catheter ablation. In general, pharmacologic therapy is associated with a ~50% recurrence rate of AF at 6-12 months (and substantial side effects), while success of single-procedure ablation is 50-70%, with non-trivial procedural risks (1/1000 risk of death). Thus, therapy targeting the electrophysiologic/anatomic basis of AF has met with only limited success. Elements of the present invention include compounds for use in treating atrial fibrillation and damage resulting therefrom. Disclosed herein is the concept that scavenging γ-KAs can prevent/treat atrial arrhythmias such as atrial fibrillation/atrial flutter. Also disclosed herein is the use of y-KA scavengers such as salicylamine and compounds of the present invention to prevent/treat other types of atrial arrhythmias (eg, atrial tachycardia).

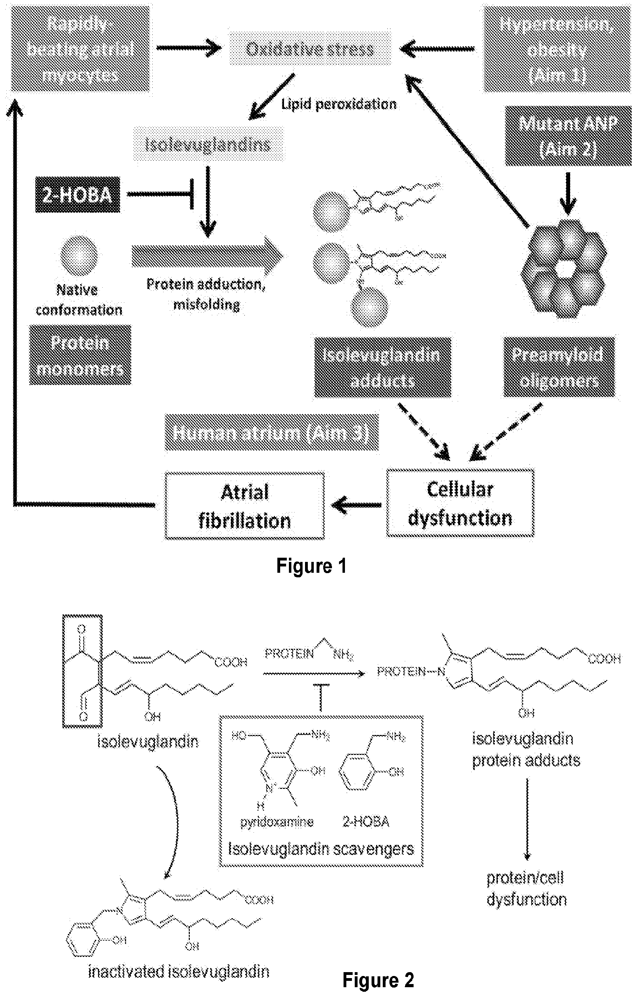

Lipid peroxidation is a major component of ROS-mediated cellular damage, and the most reactive products generated, *isolevuglandins* (IsoLGs), react almost instantaneously with proteins to cause misfolding/ dysfunction. Novel lipid dicarbonyl scavengers have been developed that preemptively bind IsoLGs before they can interact with biologic targets to damage cells, the best studied of which is 2-hydroxybenzylamine, or 2-HOBA. Recently, IsoLGs were identified as critical mediators in immune-mediated hypertension and Alzheimer's disease. For proteins that form amyloid (e.g., amyloid β₁₋₄₂ in Alzheimer's), *preamyloid oligomers* (PAOs) are now recognized to be the primary cytotoxic species that correlates with disease progression. Notably, IsoLGs markedly accelerate PAO formation for such proteins. The present inventors show that both IsoLGs and PAOs are biologically-relevant mediators that promote AF susceptibility (Figure 1), making them potential therapeutic targets.

The present inventors have identified the formation of IsoLGs and PAOs in rapidly-stimulated atrial cells, as well as models of oxidative stress-related AF susceptibility, specifically the common AF risk factors hypertension and obesity. Importantly, the data demonstrate a beneficial effect of scavenging IsoLGs to reduce AF burden, as well as oligomer formation. In human atrial tissue obtained during cardiac surgery, PAOs were frequently detected, and quantitative analysis revealed an association between atrial PAO burden and hypertension. A logical candidate for oligomer formation in the atrium is atrial natriuretic peptide (ANP), a known fibrillogenic protein encoded by the gene *NPPA.* ANP contributes to a common form of aging-related atrial amyloidosis linked to AF, and the present inventors have identified ANP in the oligomers present in human atrium. A mutation in *NPPA* that lengthens the ANP peptide is associated with familial AF. In both patients and a mouse model of this inherited disorder, AF susceptibility occurs in the absence of gross or microscopic atrial structural remodeling. The proarrhythmic mechanisms whereby mutant ANP causes AF remain uncertain. However, the present inventors found that mutant ANP is markedly more fibrillogenic than the wild-type peptide. These oligomers accumulating in the atria of mice expressing mutant ANP are cytotoxic and modulate atrial electrophysiology. Taken together, these results provide compelling evidence to support the concept that IsoLGs and PAOs are drivers of the AF substrate, constituting novel mechanisms to increase arrhythmia susceptibility.

The present inventors have also identified that in hypertension and obesity, oxidative stress-mediated isolevuglandins promotes atrial cell dysfunction/injury and AF susceptibility. The present inventors have also identified that oligomers derived from mutant ANP alter myocyte homeostasis to generate AF susceptibility, and they also promote oxidative stress/IsoLG formation that feedback in a positive manner to perpetuate the pathologic process. Additionally, the present inventors have identified that AF risk factors linked to oxidative stress increase arrhythmia susceptibility through the generation of IsoLGs and cytotoxic PAOs. Thus, the present invention addresses significant needs, given that IsoLG and PAO formation may provide not only common mechanistic links between cardiac pathophysiology and AF, but also novel therapeutic targets in the prevention and/or treatment of this common and serious arrhythmia.

*Lipid peroxidation and isolevuglandins.* One of the most susceptible sites to ROS damage is polyunsaturated fatty acids in the cell membrane and circulation. Peroxidation of these lipids generates injurious reactive aldehydes, including malondialdehyde (MDA), 4-hydroxynonenal and related 4-oxo-2-nonenal, acrolein, methylglyoxal, and isolevuglandins (IsoLGs; also called γ-ketoaldehydes or isoketals). The toxicity of such compounds is markedly augmented by the presence of 2 carbonyl groups (C=O), and the IsoLGs have a 1,4-dicarbonyl ring configuration that renders them uniquely reactive (Figure 2). These compounds react nearly instantaneously with lysine residues in proteins and thus are the most reactive products of lipid peroxidation identified to date. Indeed, they modify proteins so rapidly that they can only be detected *in vivo* as adducts rather than their unreacted form, in distinct contrast to other lipid peroxidation products. IsoLG adducts are covalent, irreversible modifications, and IsoLGs modifications include intramolecular crosslinks that cause dysfunction of proteins and structures relevant to cardiomyocyte homeostasis, including ion channels, mitochondria, and proteasomes. IsoLGs can also adduct to DNA. IsoLG adducts are increased in the diseased tissue in multiple conditions linked to oxidative injury/inflammation, and they were recently identified as critical mediators of oxidative injury in the brain in Alzheimer's disease and in the vasculature in HTN.

*Dicarbonyl scavengers to investigate mechanisms of oxidant stress-related diseases.* An obvious first approach to protect against the assault of IsoLGs and other reactive dicarbonyls would be to reduce their production. However, contemporary antioxidants have been largely ineffective to reduce ROS, or oxidative stress-related diseases in general. Currently-available antioxidants are designed to prevent ROS-mediated injury by reacting with free radicals, inhibiting the activity of free radical-generating enzymes, or enhancing activity of intracellular antioxidant enzymes. However, recent studies have shown that therapeutically-used doses of antioxidants such as vitamin E and fish oil are not effective to reduce *in vivo* measures of oxidative injury (i.e., F₂-Isoprostanes, the gold standard to measure oxidative stress). An alternative to these "upstream strategies" aimed at stopping ROS production is to leave ROS generation intact, but to rapidly scavenge the reactive dicarbonyl species as they form, so that they cannot interact with their biologic targets, thus rendering them inactive. Collaborators have identified novel aminomethylphenol compounds that react with isoLGs and thereby preemptively scavenge these isoLGs and closely-related dicarbonyls to prevent downstream protein modification. Structure activity relationship assays on the prototype pyridoxamine (Figure 2) led to generation of numerous active structural analogs with varying degrees of lipophilicity and efficacy in cellular assays. Given that dicarbonyl scavengers target downstream mediators of ROS-related injury, they represent a totally novel, alternative approach for diseases linked to oxidative stress. An embodiment of the present invention is 2-hydroxybenzylamine (2-HOBA; also called salicylamine) for use as indicated above.

Importantly, 2-HOBA is not an antioxidant (i.e., it does not reduce ROS levels significantly). Rather, it reacts with IsoLGs at a much more rapid rate (by several orders of magnitude) than IsoLGs can bind to the ε-amine of lysine. A structural requirement for this scavenging activity is the location of a hydroxyl group adjacent to a methylamine in the phenolic amine structure. For the related analog 4-HOBA, the structural proximity of these functional groups is lost - hence this compound cannot scavenge dicarbonyls and is inactive. 2-HOBA and its active analogs do not affect concentrations of O₂˙⁻ or F₂-Isoprostanes during *in vitro* oxidation, and the reduction in IsoLG adduct levels has been attributed directly to its dicarbonyl scavenging effect, and not to inhibition of ROS production and/or lipid peroxidation. An additional beneficial effect of compounds like 2-HOBA is that they can also scavenge other injurious lipid dicarbonyl compounds such as MDA, in addition to IsoLGs. However, due to their extreme reactivity based on the 1,4-dicarbonyl ring structure, IsoLGs are preferentially targeted. 2-HOBA does not inhibit the COX1 or COX2 enzymes, and thus, the production of physiologic prostaglandins is preserved. To date, 2-HOBA has been shown to prevent development of cognitive impairment in a mouse model of Alzheimer's disease, and it effectively lowered blood pressure (BP) in ang II-mediated HTN in mice. Thus, by scavenging IsoLGs preemptively, 2-HOBA and its analogs represent a paradigm shift in pharmacologic strategy to prevent injurious oxidative protein modification. 2-HOBA is a natural product with an excellent safety profile based on *in vitro* studies, *in vivo* studies in thousands of mice, and recent pre-clinical toxicology studies (Metabolic Technologies, Inc., Ames, IA).

The present inventors have discovered the role of novel mediators in the genesis of the AF substrate, thus challenging the current research paradigm for this common cardiac arrhythmia.

The present inventors have accumulated multiple lines of evidence to support these hypotheses in cells, animal models, and humans.

The present inventors have developed innovative imaging-based methods to quantitate PAO burden in small atrial samples in a reproducible manner, as a prerequisite for investigative studies in humans. The proposed experiments will establish the role of PAO-mediated proteotoxicity in promoting AF susceptibility using primarily a genetic model.

*An unexpected signal for protein misfolding.* The progressive nature of AF is caused by electrical and structural remodeling due to rapid atrial activation that increases arrhythmia susceptibility - "AF begets AF". Previously, the present inventors found that atrial cells rapidly stimulated in culture undergo remodeling similar to that observed in human AF, with striking concordance of transcriptional changes. Unexpectedly, the present inventors found conserved transcriptional up-regulation for proteins implicated in protein misfolding/amyloidosis. This finding shows that proteotoxicity, a process linked to oxidative stress, can develop in human atrium as a novel mechanism to promote atrial injury.

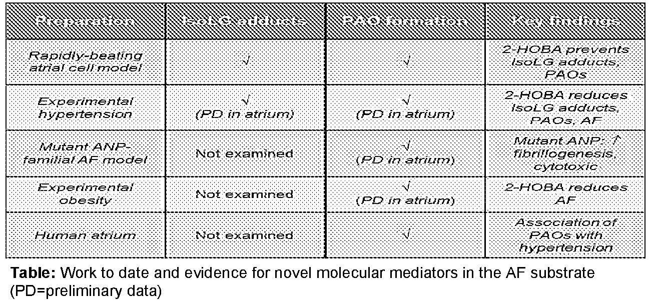

The present inventors have shown that in the setting of hypertension and obesity, oxidative stress-mediated isolevuglandins promote atrial cell dysfunction/injury and the substrate for AF. Histologic studies have demonstrated fibrosis and often atrial enlargement with established AF in the setting of HTN and obesity (with evidence of atrial contact/infiltration by pericardial fat in obesity). Nonetheless, little is known about molecular processes that lead to AF in its earliest stages.

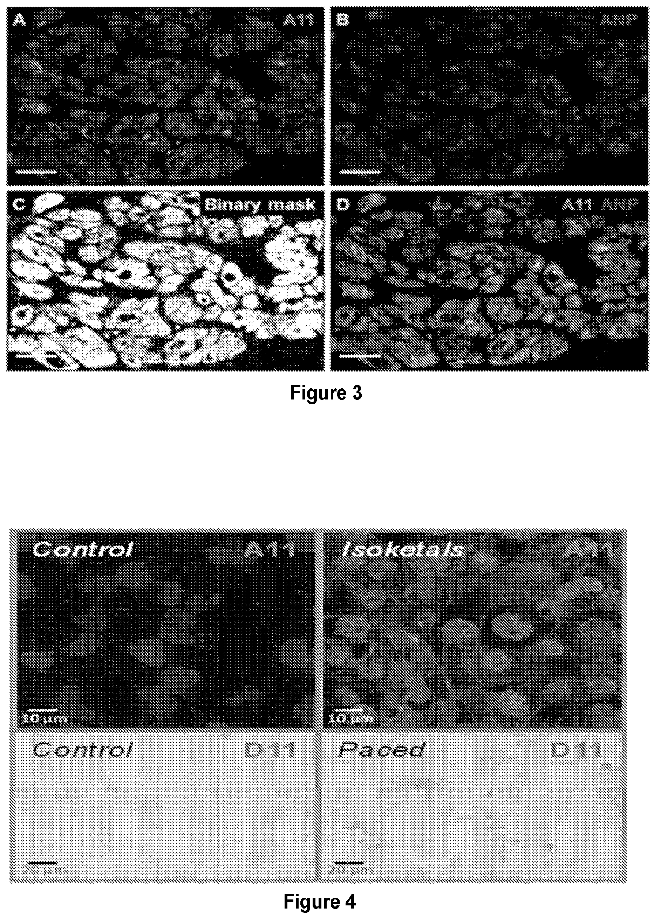

*Detection and quantitation of preamyloid oligomers in human atrium.* PAOs are not detected by Congo red. However, they share a common structural epitope irrespective of amino acid sequence, and conformation-specific antibodies, such as A-11, recognize oligomers generated by a wide variety of proteins. Using A-11 and a myocardial-specific antibody, we developed a microscopic imaging-based method to enable robust and reproducible quantitative analysis of PAO burden in atrial samples harvested at the time of elective cardiac surgery. This method quantitated the relative area of myocardium containing PAOs (Figure 3), or Green/Red ratio (G/R), as a spatial representation of PAO burden.

*Association of atrial PAOs with hypertension in patients without AF.* Using these methods, the present inventors investigated the clinical correlates of PAOs in 92 patients without a history of AF or cardiomyopathy undergoing elective cardiac surgery (mean age 61.7 years, 63% male, 72% with HTN, 42% with coronary artery disease, 67% having aortic valve replacement). Intracellular PAOs were detected in a majority of atrial samples, with ANP a significant component (Figure 3), and their presence was independent of other pathologic atrial abnormalities (e.g., fibrosis). Using a linear mixed effects model, a consistent finding across multiple analyses was the independent association of PAO burden with HTN. Given that ANP plasma concentrations are increased in experimental and human HTN, we hypothesize that elevated concentrations/oxidative stress in HTN promote misfolding of ANP to form PAOs.
*Protein oligomers and IsoLGs in a cellular model simulating AF.* The present inventors found that rapid pacing of cultured atrial HL-1 cells caused the accumulation of diffuse cytoplasmic PAOs (confirmed by Western blotting, with ANP a significant component), as well as enhanced superoxide (O₂˙⁻) production and abundant IsoLG adducts (Figure 4, lower panels, detected by an anti-IsoLG lysyl adduct antibody D11 ScFv) that were absent in control, unpaced, spontaneously-beating cells. Moreover, exposure of unpaced cells to a physiologic concentration of IsoLGs caused cytosolic PAO production, similar to that observed with rapid pacing (Figure 4, top panels). When cells were rapidly paced in presence of 2-HOBA, PAO formation was virtually eliminated, and the myocyte stress response (e.g., transcriptional upregulation of *Nppb* and *Hspala*) was blunted, indicating a cytoprotective effect of 2-HOBA.

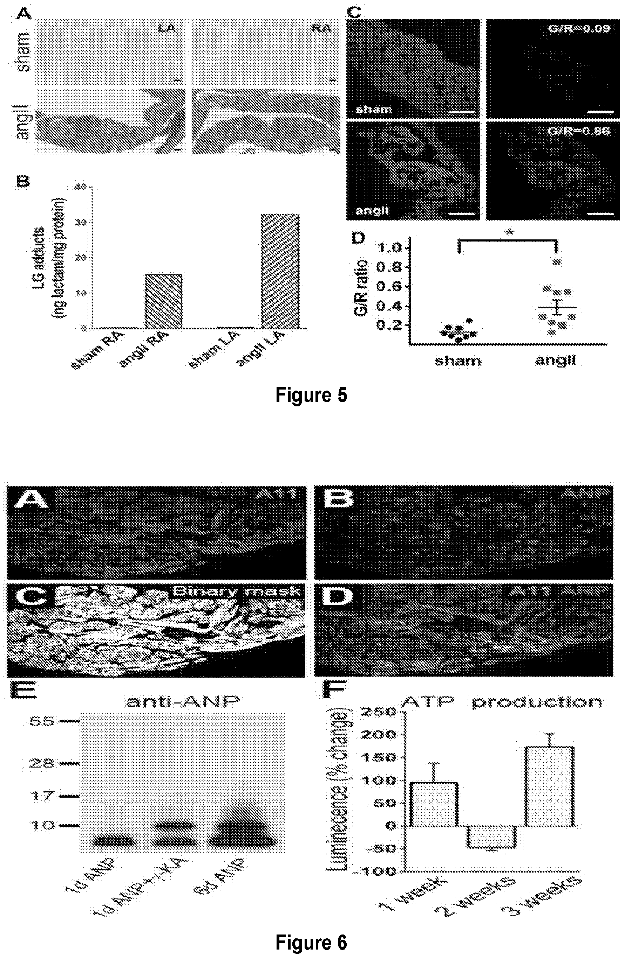

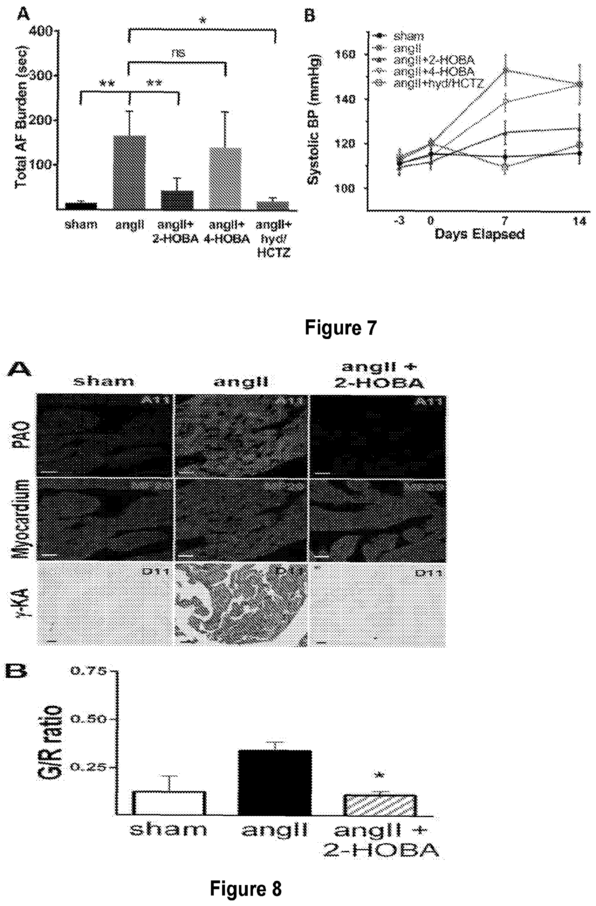

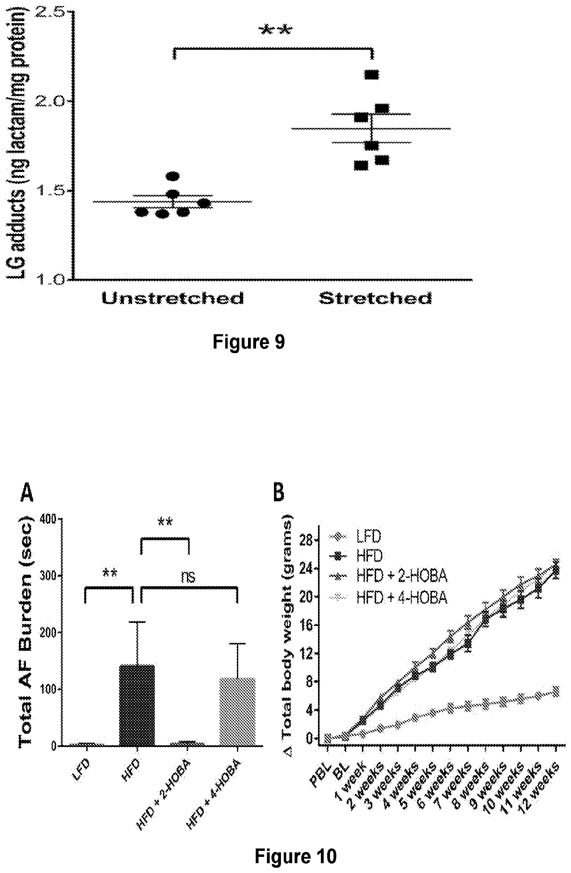

*Experimental hypertension: Evidence for IsoLGs in AF susceptibility.* Oxidative stress and PAO burden in human atrium are linked to HTN. Moreover, Kirabo and colleagues recently demonstrated the generation of IsoLG adducts in the vasculature of ang II-treated mice, and a prominent antihypertensive effect of 2-HOBA. The present inventors have determined that IsoLGs are mediators for AF susceptibility in HTN using a murine model (2wk mini-pump infusion of angiotensin II [ang II]), in which mice develop sustained HTN and inducible AF. With development of HTN, IsoLG adducts were detected in both atria by immunostaining (in the absence of fibrosis or other structural abnormalities), which did not occur with vehicle (sham) infusion (Figure 5A). Importantly, mass spectrometry data have confirmed this finding (Figure 5B), with a striking 100 fold increase in atrial adduct formation during HTN (2-3 fold greater increase than that seen in Alzheimer's brain). In addition, PAOs accumulated in the atria of hypertensive compared to control mice (Figure 5C and D). Several lines of evidence suggested a role for ANP in these oligomers: 1) immunostaining revealed evidence for partial colocalization of ANP and PAOs (Figure 6A-D); 2) IsoLGs markedly accelerated PAO formation for the ANP peptide (Figure 6E); and 3) ANP oligomers were cytotoxic (Figure 6F). As expected, hypertensive mice developed inducible AF compared to control (Figure 7). However, treatment with 2-HOBA markedly reduced AF burden, as well as atrial IsoLG adducts and PAO formation (Figure 8;1G/L in the drinking water). An inactive structural analogue, 4-HOBA, had no effect on AF susceptibility (Figure 7; 1G/L in water), strongly implying that the beneficial effect of 2-HOBA was indeed IsoLG scavenging. Normalization of BP in ang II-treated mice with hydralazine/HCTZ prevented AF susceptibility, while mechanical stretch of atrial myocytes generated both LG adducts (Figure 9) and PAOs (not shown), signifying a critical role of atriomyocyte stretch. The AF substrate was reversible by 2wks after stopping ang II (85% reduction in total AF burden), with partial normalization of BP (74%; n=13, data not shown). Of note, 2-HOBA had no effects on any ECG parameters in HTN mice. Taken together, these preliminary data strongly suggest that IsoLGs and atrial stretch play a critical role in this HTN-mediated AF substrate, providing a mechanistic link between HTN, oxidative stress, and AF susceptibility.

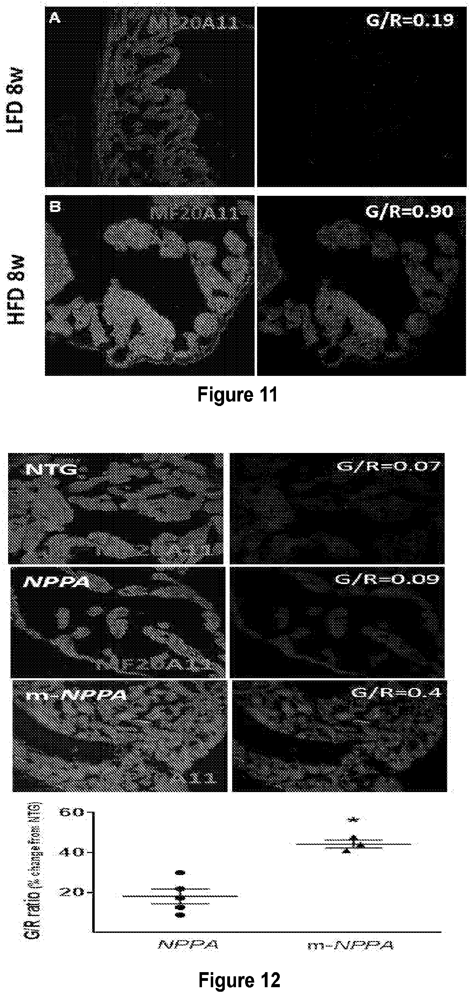

*Experimental obesity: Evidence for IsoLGs in AF susceptibility.* Given that obesity is also linked to inflammation and oxidative stress, we have begun studies to investigate the role of IsoLGs in a mouse model of diet-induced obesity (DIO). After 12 weeks of a high fat diet, obese mice displayed increased AF burden compared to lean mice (Figure 10), without evidence of atrial fibrosis, as previously reported. Importantly, co-treatment with 2-HOBA markedly suppressed inducible AF, while the inactive analogue 4-HOBA did not. Preliminary studies at 8 weeks showed that atrial PAO burden was dramatically increased (G/R=0.87; n=2) compared to the atria of lean mice (G/R=0.17; n=2; Figure 11). Thus, in the early stages of both HTN and obesity, the present inventors show a role for IsoLGs in the AF substrate. In both HTN and obesity, AF was inducible in the absence of fibrosis, with reversibility in HTN. These findings imply that IsoLGs constitute an early pathologic target, and that their inhibition could potentially prevent the development of AF. In the proposed studies, we will confirm the critical role of IsoLGs using rigorous experimental approaches. For these studies, we use well-characterized murine models of HTN and obesity described above and routinely employed. The present inventors' data indicate PAO burden and IsoLG adducts correlate with one another.

The present inventors have shown that IsoLGs are the most potent mediators of oxidative stress identified. For conditions linked to ROS such as HTN and obesity, IsoLG scavengers prevent AF susceptibility. Because IsoLGs markedly accelerate PAO formation for susceptible proteins, PAOs should develop in these ROS-mediated diseases, with prevention by IsoLG scavengers, consistent with our data to date. In obese female mice, and mice fed a high-fat oleic acid diet, IsoLG formation and AF susceptibility should be reduced, compared to traditional DIO, providing further evidence for IsoLGs in the pathophysiologic process.

Data associated with the present invention examines the role of cytotoxic mutant ANP oligomers in atrial pathophysiology and arrhythmia susceptibility for an NPPA mutation linked to familial AF. The present inventors show that mutant ANP oligomers alter atrial myocyte homeostasis to generate AF susceptibility, and they promote oxidative stress/IsoLG formation that feedback in a positive manner to perpetuate the pathologic process.

Fibrillogenic proteins like ANP are diverse and unrelated in their primary amino acid structure. Factors that contribute to fibrillogenesis include variant protein structure, extensive β conformation of the precursor protein, and proteolytic processing of the precursor protein (as for amyloid β₁₋₄₂). In many cases, amyloidosis occurs as a consequence of a mutation or modification in the primary structure of a causative protein, or mutations/conditions causing its overproduction.

The gene NPPA encodes the precursor prepro-ANP, which undergoes proteolytic processing to generate N-terminal pro-ANP and ANP. Genetic studies have linked abnormal ANP production to familial AF. In a large family with Holt-Oram Syndrome, a missense mutation in T-box transcription factor 5 (TBx5) resulted in an atypical phenotype with early-onset AF and the overexpression of multiple genes, including NPPA. Familial AF has also been linked to mutations in NPPA itself. The best-studied example derives from a large family having multiple affected members with early-onset lone AF. A 2-base pair deletion was identified that abolished the normal stop codon, leading to a mature ANP protein containing the usual 28 amino acids plus an anomalous C-terminus of 12 additional residues. Plasma concentrations of mutant ANP in affected family members were 5-10 times higher than wild-type (WT) ANP due to abnormal proteolytic degradation. In affected patients, AF susceptibility occurred in the absence of gross atrial structural remodeling. Electrophysiologic mechanisms have been proposed to explain the pathogenesis of this mutation, but findings are controversial. The present inventors show that for mutant ANP, the altered amino acid sequence leads to accelerated protein misfolding and PAO development in the atria, as the proximal mechanism to increase arrhythmia susceptibility. Development of atrial PAOs in mice expressing mutant ANP. Recently, in the laboratory of Dr. Dawood Darbar, transgenic mice were generated that overexpress wild-type (WT) human ANP, or human ANP harboring the frame-shift mutation described above. These mice demonstrated phenotypic features reflective of the modeled human disease. Atrial volumes and left ventricular ejection fraction in the transgenic mice were similar to those of non-transgenic (NTG) animals (with a lower BP). For mice expressing mutant ANP, levels of circulating mutant ANP were elevated 5-6 fold (as in humans), and inducible AF was significantly increased, compared to WT-ANP or nontransgenic (NTG) mice. We show that mutant ANP promotes PAO accumulation in the atrium to increase AF susceptibility. Preliminary immunostaining revealed robust accumulation of PAOs in the atria of mutant ANP mice compared to NTG and WT-ANP mice (Figure 12). These data support the concept of PAO formation as a driver of the AF substrate.

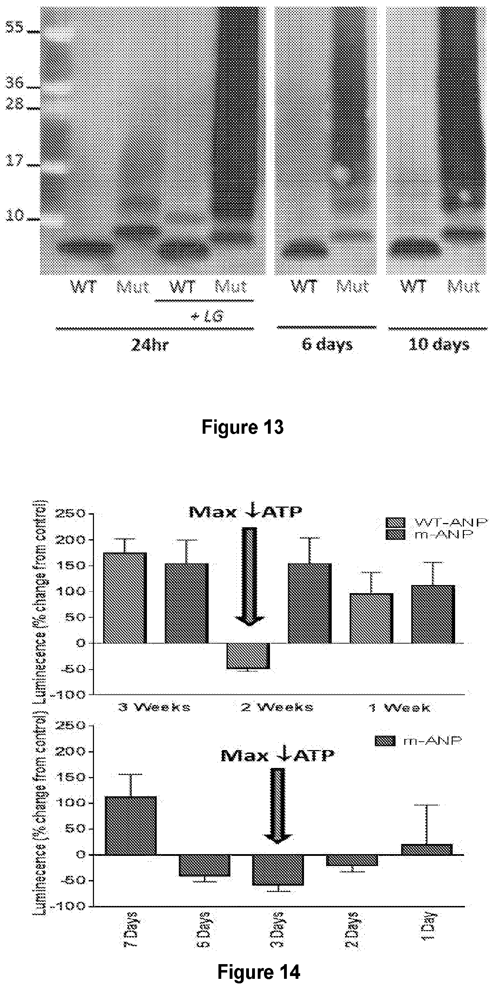

Mutant ANP is highly fibrillogenic, generating cytotoxic PAOs. To compare oligomer formation between WT and mutant ANP, Western analysis was performed on peptides allowed to oligomerize at 23°C for variable time points. WT-ANP displayed time- and IsoLG-dependent fibrillogenesis; however, this process was markedly accelerated for the mutant peptide (Figure 13). For amyloid β₁₋₄₂, oligomer toxicity is a transient, time-dependent phenomenon, in the progression from monomers to PAOs to fibrils. To investigate this process for WT and mutant ANP, peptides incubated in vitro to generate oligomers over different time periods were incubated with atrial HL-1 cells for 24hr. For WT ANP, proteotoxicity (manifested by decreased cellular ATP production) was maximal for PAOs generated during a 2wk incubation, while for mutant ANP, the time point of maximal oligomer cytotoxicity occurred much earlier, within ~3d (Figure 14). Cytotoxicity was confirmed by measuring cellular oxygen consumption rate using extracellular flux analysis (Seahorse Bioscience XFE96). Thus, oligomers formed by both WT and mutant ANP cause atrial myocyte dysfunction, as evidenced by reduced cardiomyocyte ATP production, with proteotoxicity occurring earlier for mutant compared to WT ANP, consistent with accelerated oligomer formation.

*Mutant ANP PAOs cause potentially proarrhythmic electrophysiologic effects in atrial cells.* Peptides were incubated in PBS for 24h: at this time point, mutant ANP develops oligomers but WT ANP does not (Figure 13).

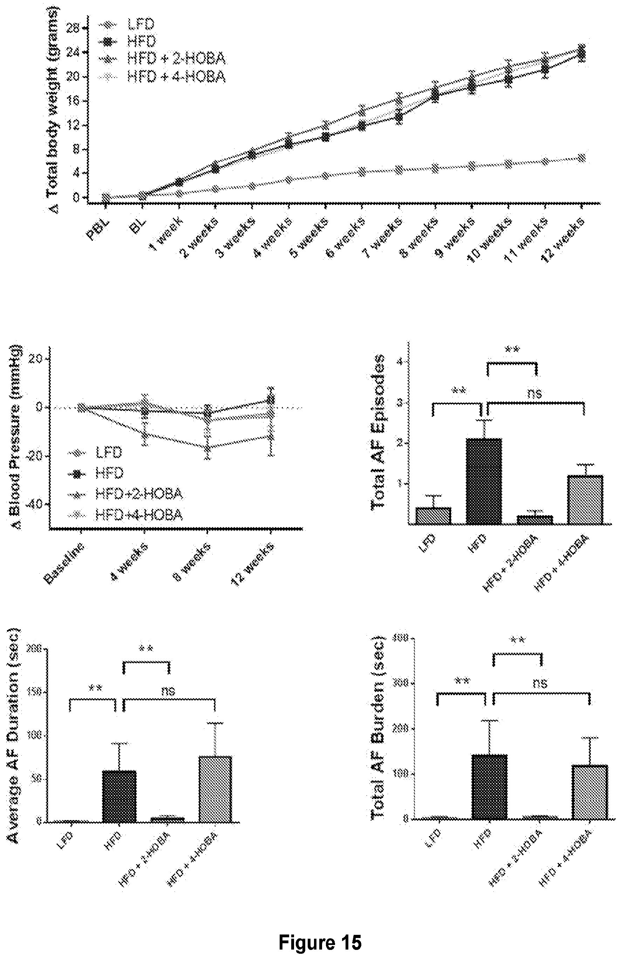

Figure 15 is an example that shows a diet-induced obesity model. The following data are for 12 week-treated animals. Total AF (atrial fibrillation) burden is probably the best measure - the AF burden for animals treated with 2-HOBA was similar to that for the lean (low fat diet) mice, while 4-HOBA had no effect.

### REFERENCES

Publications cited herein, include the following:
Patel NJ, Deshmukh A, Pant S, Singh V, Patel N, Arora S, Shah N, Chothani A, Savani GT, Mehta K, Parikh V, Rathod A, Badheka AO, Lafferty J, Kowalski M, Mehta JL, Mitrani RD, Viles-Gonzalez JF, Paydak H. Contemporary trends of hospitalization for atrial fibrillation in the United States, 2000 through 2010: implications for healthcare planning. Circulation 2014 June 10;129(23):2371-9.
Ball J, Carrington MJ, McMurray JJ, Stewart S. Atrial fibrillation: profile and burden of an evolving epidemic in the 21st century. Int J Cardiol 2013 September 1;167(5):1807-24.
Chugh SS, Havmoeller R, Narayanan K, Singh D, Rienstra M, Benjamin EJ, Gillum RF, Kim YH, McAnulty JH, Jr., Zheng ZJ, Forouzanfar MH, Naghavi M, Mensah GA, Ezzati M, Murray CJ. Worldwide epidemiology of atrial fibrillation: a Global Burden of Disease 2010 Study. Circulation 2014 February 25;129(8):837-47.
January CT, Wann LS, Alpert JS, Calkins H, Cleveland JC, Jr., Cigarroa JE, Conti JB, Ellinor PT, Ezekowitz MD, Field ME, Murray KT, Sacco RL, Stevenson WG, Tchou PJ, Tracy CM, Yancy CW. 2014 AHA/ACC/HRS Guideline for the Management of Patients With Atrial Fibrillation: A Report of the American College of Cardiology/American Heart Association Task Force on Practice Guidelines and the Heart Rhythm Society. Circulation 2014 April 10.
Shoemaker MB, Hemnes AR, Robbins IM, Langberg JJ, Ellis CR, Aznaurov SG, Fredi JL, Slosky DA, Roden DM, Murray KT, Piana RN, Mendes LA, Whalen SP. Left atrial hypertension after repeated catheter ablations for atrial fibrillation. J Am Coll Cardiol 2011 May 10;57(19):1918-9.
Gupta A, Perera T, Ganesan A, Sullivan T, Lau DH, Roberts-Thomson KC, Brooks AG, Sanders P. Complications of catheter ablation of atrial fibrillation: a systematic review. Circ Arrhythm Electrophysiol 2013 December;6(6):1082-8.
Iwasaki YK, Nishida K, Kato T, Nattel S. Atrial fibrillation pathophysiology: implications for management. Circulation 2011 November 15;124(20):2264-74.
Chimenti C, Russo MA, Carpi A, Frustaci A. Histological substrate of human atrial fibrillation. Biomed Pharmacother 2010 March;64(3):177-83.
Wanahita N, Messerli FH, Bangalore S, Gami AS, Somers VK, Steinberg JS. Atrial fibrillation and obesity--results of a meta-analysis. Am Heart J 2008 February;155(2):310-5.
Nalliah CJ, Sanders P, Kottkamp H, Kalman JM. The role of obesity in atrial fibrillation. Eur Heart J 2015 September 14.
Abed HS, Samuel CS, Lau DH, Kelly DJ, Royce SG, Alasady M, Mahajan R, Kuklik P, Zhang Y, Brooks AG, Nelson AJ, Worthley SG, Abhayaratna WP, Kalman JM, Wittert GA, Sanders P. Obesity results in progressive atrial structural and electrical remodeling: implications for atrial fibrillation. Heart Rhythm 2013 January;10(1):90-100.
Mahajan R, Lau DH, Brooks AG, Shipp NJ, Manavis J, Wood JP, Finnie JW, Samuel CS, Royce SG, Twomey DJ, Thanigaimani S, Kalman JM, Sanders P. Electrophysiological, Electroanatomical, and Structural Remodeling of the Atria as Consequences of Sustained Obesity. J Am Coll Cardiol 2015 July 7;66(1):1-11.
Murphy MP. How mitochondria produce reactive oxygen species. Biochem J 2009 January 1;417(1):1-13.
Stadtman ER, Berlett BS. Reactive oxygen-mediated protein oxidation in aging and disease. Drug Metab Rev 1998 May;30(2):225-43.
Sayre LM, Smith MA, Perry G. Chemistry and biochemistry of oxidative stress in neurodegenerative disease. Curr Med Chem 2001 June;8(7):721-38.
Groeger AL, Freeman BA. Signaling actions of electrophiles: anti-inflammatory therapeutic candidates. Mol Interv 2010 February;10(1):39-50.
Sugamura K, Keaney JF, Jr. Reactive oxygen species in cardiovascular disease. Free Radic Biol Med 2011 September 1;51(5):978-92.
Brown DI, Griendling KK. Regulation of signal transduction by reactive oxygen species in the cardiovascular system. Circ Res 2015 January 30;116(3):531-49.
Gutierrez A, Van Wagoner DR. Oxidant and Inflammatory Mechanisms and Targeted Therapy in Atrial Fibrillation: An Update. J Cardiovasc Pharmacol 2015 December;66(6):523-9.
De Jong AM, Maass AH, Oberdorf-Maass SU, Van Veldhuisen DJ, Van Gilst WH, Van Gelder IC. Mechanisms of atrial structural changes caused by stretch occurring before and during early atrial fibrillation. Cardiovasc Res 2011 March 1;89(4):754-65.
Jacob KA, Nathoe HM, Dieleman JM, van OD, Kluin J, van DD. Inflammation in new-onset atrial fibrillation after cardiac surgery: a systematic review. Eur J Clin Invest 2014 April;44(4):402-28.
Mihm MJ, Yu F, Carnes CA, Reiser PJ, McCarthy PM, Van Wagoner DR, Bauer JA. Impaired myofibrillar energetics and oxidative injury during human atrial fibrillation. Circulation 2001 July 10;104(2):174-80.
Kim YM, Guzik TJ, Zhang YH, Zhang MH, Kattach H, Ratnatunga C, Pillai R, Channon KM, Casadei B. A myocardial Nox2 containing NAD(P)H oxidase contributes to oxidative stress in human atrial fibrillation. Circ Res 2005 September 30;97(7):629-36.
Dudley SC, Jr., Hoch NE, McCann LA, Honeycutt C, Diamandopoulos L, Fukai T, Harrison DG, Dikalov SI, Langberg J. Atrial fibrillation increases production of superoxide by the left atrium and left atrial appendage: role of the NADPH and xanthine oxidases. Circulation 2005 August 30;112(9):1266-73.
Savelieva I, Kakouros N, Kourliouros A, Camm AJ. Upstream therapies for management of atrial fibrillation: review of clinical evidence and implications for European Society of Cardiology guidelines. Part I: primary prevention. Europace 2011 March;13(3):308-28.
Savelieva I, Kakouros N, Kourliouros A, Camm AJ. Upstream therapies for management of atrial fibrillation: review of clinical evidence and implications for European Society of Cardiology guidelines. Part II: secondary prevention. Europace 2011 May;13(5):610-25.
Salomon RG, Miller DB, Zagorski MG, Coughlin DJ. Solvent induced fragmentation of prostaglandin endoperoxides. New aldehyde products from PGH2 and novel intramolecular 1,2-hydride shift during endoperoxide fragmentation in aqueous solution. J Am Chem Soc 1984;106:6049-60.
Salomon RG, Subbanagounder G, O'Neil J, Kaur K, Smith MA, Hoff HF, Perry G, Monnier VM. Levuglandin E2-protein adducts in human plasma and vasculature. Chem Res Toxicol 1997 May;10(5):536-45.
Salomon RG, Batyreva E, Kaur K, Sprecher DL, Schreiber MJ, Crabb JW, Penn MS, DiCorletoe AM, Hazen SL, Podrez EA. Isolevuglandin-protein adducts in humans: products of free radical-induced lipid oxidation through the isoprostane pathway. Biochim Biophys Acta 2000 May 31;1485(2-3):225-35.
Brame CJ, Salomon RG, Morrow JD, Roberts LJ. Identification of extremely reactive gamma-ketoaldehydes (isolevuglandins) as products of the isoprostane pathway and characterization of their lysyl protein adducts. J Biol Chem 1999 May 7;274(19):13139-46.
Brame CJ, Boutaud O, Davies SS, Yang T, Oates JA, Roden D, Roberts LJ. Modification of proteins by isoketal-containing oxidized phospholipids. J Biol Chem 2004 April 2;279(14):13447-51.
Amamath V, Amamath K, Amarnath K, Davies S, Roberts LJ. Pyridoxamine: an extremely potent scavenger of 1,4-dicarbonyls. Chem Res Toxicol 2004 March;17(3):410-5.
Fukuda K, Davies SS, Nakajima T, Ong BH, Kupershmidt S, Fessel J, Amarnath V, Anderson ME, Boyden PA, Viswanathan PC, Roberts LJ, Balser JR. Oxidative mediated lipid peroxidation recapitulates proarrhythmic effects on cardiac sodium channels. Circ Res 2005 December 9;97(12):1262-9.
Nakajima T, Davies SS, Matafonova E, Potet F, Amarnath V, Tallman KA, Serwa RA, Porter NA, Balser JR, Kupershmidt S, Roberts LJ, III. Selective γ-ketoaldehyde scavengers protect Nav1.5 from oxidant-induced inactivation. J Mol Cell Cardiol 2010 February;48(2):352-9.
Stavrovskaya IG, Baranov SV, Guo X, Davies SS, Roberts LJ, Kristal BS. Reactive γ-ketoaldehydes formed via the isoprostane pathway disrupt mitochondrial respiration and calcium homeostasis. Free Radic Biol Med 2010 August 15;49(4):567-79.
Davies SS, Amamath V, Montine KS, Bernoud-Hubac N, Boutaud O, Montine TJ, Roberts LJ. Effects of reactive γ-ketoaldehydes formed by the isoprostane pathway (isoketals) and cyclooxygenase pathway (levuglandins) on proteasome function. FASEB J 2002 May;16(7):715-7.
Carrier EJ, Amamath V, Oates JA, Boutaud O. Characterization of covalent adducts of nucleosides and DNA formed by reaction with levuglandin. Biochemistry 2009 November 17;48(45):10775-81.
Davies SS, Bodine C, Matafonova E, Pantazides BG, Bernoud-Hubac N, Harrison FE, Olson SJ, Montine TJ, Amamath V, Roberts LJ. Treatment with a γ-ketoaldehyde scavenger prevents working memory deficits in hApoE4 mice. J Alzheimers Dis 2011;27(1):49-59.
Kirabo A, Fontana V, de Faria AP, Loperena R, Galindo CL, Wu J, Bikineyeva AT, Dikalov S, Xiao L, Chen W, Saleh MA, Trott DW, Itani HA, Vinh A, Amamath V, Amamath K, Guzik TJ, Bernstein KE, Shen XZ, Shyr Y, Chen SC, Memaugh RL, Laffer CL, Elijovich F, Davies SS, Moreno H, Madhur MS, Roberts J, Harrison DG. DC isoketal-modified proteins activate T cells and promote hypertension. J Clin Invest 2014 October 1;124(10):4642-56.
Lu JM, Lin PH, Yao Q, Chen C. Chemical and molecular mechanisms of antioxidants: experimental approaches and model systems. J Cell Mol Med 2010 April;14(4):840-60.
Roberts LJ, Oates JA, Linton MF, Fazio S, Meador BP, Gross MD, Shyr Y, Morrow JD. The relationship between dose of vitamin E and suppression of oxidative stress in humans. Free Radic Biol Med 2007 November 15;43(10):1388-93.
Darghosian L, Free M, Li J, Gebretsadik T, Bian A, Shintani A, McBride BF, Solus J, Milne G, Crossley GH, Thompson D, Vidaillet H, Okafor H, Darbar D, Murray KT, Stein CM. Effect of omega-three polyunsaturated fatty acids on inflammation, oxidative stress, and recurrence of atrial fibrillation. Am J Cardiol 2015 January 15;115(2):196-201.
Davies SS, Brantley EJ, Voziyan PA, Amamath V, Zagol-Ikapitte I, Boutaud O, Hudson BG, Oates JA, Roberts LJ. Pyridoxamine analogues scavenge lipid-derived gamma-ketoaldehydes and protect against H2O2-mediated cytotoxicity. Biochemistry 2006 December 26;45(51):15756-67.
Zagol-Ikapite I, Sosa IR, Oram D, Judd A, Amamath K, Amamath V, Stec D, Oates JA, Boutaud O. Modification of platelet proteins by malondialdehyde: prevention by dicarbonyl scavengers. J Lipid Res 2015 November;56(11):2196-205.
Amamath V, Amamath K. Scavenging 4-Oxo-2-nonenal. Chem Res Toxicol 2015 October 19;28(10):1888-90.
Amamath V, Amamath K, Avance J, Stec DF, Voziyan P. 5'-O-Alkylpyridoxamines: Lipophilic Analogues of Pyridoxamine Are Potent Scavengers of 1,2-Dicarbonyls. Chem Res Toxicol 2015 July 20;28(7):1469-75.
Zagol-Ikapitte I, Amamath V, Bala M, Roberts LJ, Oates JA, Boutaud O. Characterization of scavengers of γ-ketoaldehydes that do not inhibit prostaglandin biosynthesis. Chem Res Toxicol 2010 January;23(1):240-50.
Willis MS, Patterson C. Proteotoxicity and cardiac dysfunction--Alzheimer's disease of the heart? N Engl J Med 2013 January 31;368(5):455-64.
Klein WL, Krafft GA, Finch CE. Targeting small Aβ oligomers: the solution to an Alzheimer's disease conundrum? Trends Neurosci 2001 April;24(4):219-24.
Glabe CG, Kayed R. Common structure and toxic function of amyloid oligomers implies a common mechanism of pathogenesis. Neurology 2006 January 24;66(2 Suppl 1):S74-S78.
Guerrero-Munoz MJ, Castillo-Carranza DL, Kayed R. Therapeutic approaches against common structural features of toxic oligomers shared by multiple amyloidogenic proteins. Biochem Pharmacol 2014 April 15;88(4):468-78.
McLendon PM, Robbins J. Desmin-related cardiomyopathy: an unfolding story. Am J Physiol Heart Circ Physiol 2011 October;301(4):H1220-H1228.
Boutaud O, Ou JJ, Chaurand P, Caprioli RM, Montine TJ, Oates JA. Prostaglandin H2 (PGH2) accelerates formation of amyloid β1-42 oligomers. J Neurochem 2002 August;82(4):1003-6.
Pattison JS, Sanbe A, Maloyan A, Osinska H, Klevitsky R, Robbins J. Cardiomyocyte expression of a polyglutamine preamyloid oligomer causes heart failure. Circulation 2008 May 27;117(21):2743-51.
Mielcarek M, Inuabasi L, Bondulich MK, Muller T, Osborne GF, Franklin SA, Smith DL, Neueder A, Rosinski J, Rattray I, Protti A, Bates GP. Dysfunction of the CNS-heart axis in mouse models of Huntington's disease. PLoS Genet 2014 August;10(8):e1004550.
Wang X, Osinska H, Klevitsky R, Gerdes AM, Nieman M, Lorenz J, Hewett T, Robbins J. Expression of R120G-αB-crystallin causes aberrant desmin and αB-crystallin aggregation and cardiomyopathy in mice. Circ Res 2001 July 6;89(1):84-91.
Meehan S, Knowles TP, Baldwin AJ, Smith JF, Squires AM, Clements P, Treweek TM, Ecroyd H, Tartaglia GG, Vendruscolo M, Macphee CE, Dobson CM, Carver JA. Characterisation of amyloid fibril formation by small heat-shock chaperone proteins human αA-, αB- and R120G αB-crystallins. J Mol Biol 2007 September 14;372(2):470-84.
Sanbe A, Osinska H, Saffitz JE, Glabe CG, Kayed R, Maloyan A, Robbins J. Desmin-related cardiomyopathy in transgenic mice: a cardiac amyloidosis. Proc Natl Acad Sci U S A 2004 July 6;101(27):10132-6.
Gianni D, Li A, Tesco G, McKay KM, Moore J, Raygor K, Rota M, Gwathmey JK, Dec GW, Aretz T, Leri A, Semigran MJ, Anversa P, Macgillivray TE, Tanzi RE, del MF. Protein aggregates and novel presenilin gene variants in idiopathic dilated cardiomyopathy. Circulation 2010 March 16;121(10):1216-26.
Rocken C, Peters B, Juenemann G, Saeger W, Klein HU, Huth C, Roessner A, Goette A. Atrial amyloidosis: an arrhythmogenic substrate for persistent atrial fibrillation. Circulation 2002 October 15;106(16):2091-7.
Steiner I, Hajkova P. Patterns of isolated atrial amyloid: a study of 100 hearts on autopsy. Cardiovasc Pathol 2006 September;15(5):287-90.
Leone O, Boriani G, Chiappini B, Pacini D, Cenacchi G, Martin SS, Rapezzi C, Bacchi Reggiani ML, Marinelli G. Amyloid deposition as a cause of atrial remodelling in persistent valvular atrial fibrillation. Eur Heart J 2004 July;25(14):1237-41.
Johansson B, Wemstedt C, Westermark P. Atrial natriuretic peptide deposited as atrial amyloid fibrils. Biochem Biophys Res Commun 1987 November 13;148(3):1087-92.
Prinsen JK, Savio-Galimberti E, Yermalitskaya LV, Sidorova TN, Barnett JV, Boutaud OG, Darbar D, Murray KT. A frame-shift mutation of NPPA promotes formation of cytotoxic preamyloid oligomers by mutant atrial natriuretic peptide. Heart Rhythm 12 (5S), -S4. 2015.
Wijffels MC, Kirchhof CJ, Dorland R, Allessie MA. Atrial fibrillation begets atrial fibrillation. A study in awake chronically instrumented goats. Circulation 1995;92(7):1954-68.
Mace LC, Yermalitskaya LV, Yi Y, Yang Z, Morgan AM, Murray KT. Transcriptional remodeling of rapidly stimulated HL-1 atrial myocytes exhibits concordance with human atrial fibrillation. J Mol Cell Cardiol 2009 October;47(4):485-92.
Andrade J, Khairy P, Dobrev D, Nattel S. The clinical profile and pathophysiology of atrial fibrillation: relationships among clinical features, epidemiology, and mechanisms. Circ Res 2014 April 25;114(9):1453-68.
Kayed R, Head E, Thompson JL, McIntire TM, Milton SC, Cotman CW, Glabe CG. Common structure of soluble amyloid oligomers implies common mechanism of pathogenesis. Science 2003 April 18;300(5618):486-9.
Sidorova TN, Mace LC, Wells KS, Yermalitskaya LV, Su PF, Shyr Y, Byrne JG, Petracek MR, Greelish JP, Hoff SJ, Ball SK, Glabe CG, Brown NJ, Barnett JV, Murray KT. Quantitative imaging of preamyloid oligomers, a novel structural abnormality, in human atrial samples. J Histochem Cytochem 2014 April 30;62(7):479-87.
Sidorova TN, Mace LC, Wells KS, Yermalitskaya LV, Su PF, Shyr Y, Atkinson JB, Fogo AB, Prinsen JK, Byrne JG, Petracek MR, Greelish JP, Hoff SJ, Ball SK, Glabe CG, Brown NJ, Barnett JV, Murray KT. Hypertension is associated with preamyloid oligomers in human atrium: a missing link in atrial pathophysiology? J Am Heart Assoc 2014 December;3(6):e001384.
Mozaffarian D, Marchioli R, Macchia A, Silletta MG, Ferrazzi P, Gardner TJ, Latini R, Libby P, Lombardi F, O'Gara PT, Page RL, Tavazzi L, Tognoni G. Fish oil and postoperative atrial fibrillation: the Omega-3 Fatty Acids for Prevention of Post-operative Atrial Fibrillation (OPERA) randomized trial. JAMA 2012 November 21;308(19):2001-11.
Marttila M, Vuolteenaho O, Ganten D, Nakao K, Ruskoaho H. Synthesis and secretion of natriuretic peptides in the hypertensive TGR(mREN-2)27 transgenic rat. Hypertension 1996 December;28(6):995-1004.
Kawakami H, Okayama H, Hamada M, Hiwada K. Alteration of atrial natriuretic peptide and brain natriuretic peptide gene expression associated with progression and regression of cardiac hypertrophy in renovascular hypertensive rats. Clin Sci (Lond) 1996 March;90(3):197-204.
Kuroski de Bold ML. Atrial natriuretic factor and brain natriuretic peptide gene expression in the spontaneous hypertensive rat during postnatal development. Am J Hypertens 1998 August;11(8 Pt 1):1006-18.
Sergeeva IA, Christoffels VM. Regulation of expression of atrial and brain natriuretic peptide, biomarkers for heart development and disease. Biochim Biophys Acta 2013 December;1832(12):2403-13.
Sidorova TN, Yermalitskaya LV, Mace LC, Wells KS, Boutaud O, Prinsen JK, Davies SS, Roberts LJ, Dikalov SI, Glabe CG, Amamath V, Barnett JV, Murray KT. Reactive γ-ketoaldehydes promote protein misfolding and preamyloid oligomer formation in rapidly-activated atrial cells. J Mol Cell Cardiol 2015;79:295-302.
Fukui A, Takahashi N, Nakada C, Masaki T, Kume O, Shinohara T, Teshima Y, Hara M, Saikawa T. Role of leptin signaling in the pathogenesis of angiotensin II-mediated atrial fibrosis and fibrillation. Circ Arrhythm Electrophysiol 2013 April;6(2):402-9.
Purohit A, Rokita AG, Guan X, Chen B, Koval OM, Voigt N, Neef S, Sowa T, Gao Z, Luczak ED, Stefansdottir H, Behunin AC, Li N, El-Accaoui RN, Yang B, Swaminathan PD, Weiss RM, Wehrens XH, Song LS, Dobrev D, Maier LS, Anderson ME. Oxidized Ca2+/calmodulin-dependent protein kinase II triggers atrial fibrillation. Circulation 2013 October 15;128(16):1748-57.
Wu J, Thabet SR, Kirabo A, Trott DW, Saleh MA, Xiao L, Madhur MS, Chen W, Harrison DG. Inflammation and mechanical stretch promote aortic stiffening in hypertension through activation of p38 mitogen-activated protein kinase. Circ Res 2014 February 14;114(4):616-25.
Lob HE, Schultz D, Marvar PJ, Davisson RL, Harrison DG. Role of the NADPH oxidases in the subfomical organ in angiotensin II-induced hypertension. Hypertension 2013 February;61(2):382-7.
Dikalova AE, Bikineyeva AT, Budzyn K, Nazarewicz RR, McCann L, Lewis W, Harrison DG, Dikalov SI. Therapeutic targeting of mitochondrial superoxide in hypertension. Circ Res 2010 July 9;107(1):106-16.
Prinsen JK, Sidorova TN, Yermaliskaya LV, Norlander AE, Kirabo, Madhur MS, Barnett JV, Boutaud O, Kannankeril PJ, Harrison DG, Murray KT. Reactive γ-ketoaldehydes promote protein misfolding and atrial arrhythmia susceptibility in experimental hypertension. Heart Rhythm 13, S284. 2016.
Zagol-Ikapitte I, Masterson TS, Amarnath V, Montine TJ, Andreasson KI, Boutaud O, Oates JA. Prostaglandin H2-derived adducts of proteins correlate with Alzheimer's disease severity. J Neurochem 2005 August;94(4):1140-5.
Anderson EK, Gutierrez DA, Kennedy A, Hasty AH. Weight cycling increases T-cell accumulation in adipose tissue and impairs systemic glucose tolerance. Diabetes 2013 September;62(9):3180-8.
Kennedy A, Webb CD, Hill AA, Gruen ML, Jackson LG, Hasty AH. Loss of CCR5 results in glucose intolerance in diet-induced obese mice. Am J Physiol Endocrinol Metab 2013 October 1;305(7):E897-E906.
Orr JS, Kennedy A, Anderson-Baucum EK, Webb CD, Fordahl SC, Erikson KM, Zhang Y, Etzerodt A, Moestrup SK, Hasty AH. Obesity alters adipose tissue macrophage iron content and tissue iron distribution. Diabetes 2014 February;63(2):421-32.
Savio-Galimberti E, Kannankeril PJ, Stevenson TD, Kor K, Wasserman D, Darbar D. Reversal of atrial fibrillation inducibility and buren after weight loss in an obesity mouse model. Heart Rhythm 12[(5S)], -S3. 2015.
Boutaud O, Brame CJ, Chaurand P, Li J, Rowlinson SW, Crews BC, Ji C, Marnett LJ, Caprioli RM, Roberts LJ, Oates JA. Characterization of the lysyl adducts of prostaglandin H-synthases that are derived from oxygenation of arachidonic acid. Biochemistry 2001 June 12;40(23):6948-55.
Carrier EJ, Zagol-Ikapitte I, Amarnath V, Boutaud O, Oates JA. Levuglandin forms adducts with histone H4 in a cyclooxygenase-2-dependent manner, altering its interaction with DNA. Biochemistry 2014 April 22;53(15):2436-41.
Davies SS, Amamath V, Brame CJ, Boutaud O, Roberts LJ. Measurement of chronic oxidative and inflammatory stress by quantification of isoketal/levuglandin γ-ketoaldehyde protein adducts using liquid chromatography tandem mass spectrometry. Nat Protoc 2007;2(9):2079-91.
Xue B, Pamidimukkala J, Hay M. Sex differences in the development of angiotensin II-induced hypertension in conscious mice. Am J Physiol Heart Circ Physiol 2005 May;288(5):H2177-H2184.
De Jong AM, Maass AH, Oberdorf-Maass SU, De Boer RA, Van Gilst WH, Van Gelder IC. Cyclical stretch induces structural changes in atrial myocytes. J Cell Mol Med 2013 June;17(6):743-53.
Kocalis H, Niswender KD. Effects of different types of dietary fats on the cardiometabolic phenotype of rats consuming an obesogenic, high-fat, high-sucrose diet. Submitted, Annual Meeting of the ADA . 2016.
Simonds SE, Pryor JT, Ravussin E, Greenway FL, Dileone R, Allen AM, Bassi J, Elmquist JK, Keogh JM, Henning E, Myers MG, Jr., Licinio J, Brown RD, Enriori PJ, O'Rahilly S, Sternson SM, Grove KL, Spanswick DC, Farooqi IS, Cowley MA. Leptin mediates the increase in blood pressure associated with obesity. Cell 2014 December 4;159(6):1404-16.
Kay E, Gomez-Garcia L, Woodfin A, Scotland RS, Whiteford JR. Sexual dimorphisms in leukocyte trafficking in a mouse peritonitis model. J Leukoc Biol 2015 November;98(5):805-17.
Pettersson US, Walden TB, Carlsson PO, Jansson L, Phillipson M. Female mice are protected against high-fat diet induced metabolic syndrome and increase the regulatory T cell population in adipose tissue. PLoS One 2012;7(9):e46057.
Singer K, Maley N, Mergian T, DelProposto J, Cho KW, Zamarron BF, Martinez-Santibanez G, Geletka L, Muir L, Wachowiak P, Demirjian C, Lumeng CN. Differences in Hematopoietic Stem Cells Contribute to Sexually Dimorphic Inflammatory Responses to High Fat Diet-induced Obesity. J Biol Chem 2015 May 22;290(21):13250-62.
Oliveira V, Marinho R, Vitorino D, Santos GA, Moraes JC, Dragano N, Sartori-Cintra A, Pereira L, Catharino RR, da Silva AS, Ropelle ER, Pauli JR, De Souza CT, Velloso LA, Cintra DE. Diets Containing alpha-Linolenic (omega3) or Oleic (omega9) Fatty Acids Rescues Obese Mice From Insulin Resistance. Endocrinology 2015 November;156(11):4033-46.
Priori SG, Napolitano C, Tiso N, Memmi M, Vignati G, Bloise R, Sorrentino V, Danieli GA. Mutations in the cardiac ryanodine receptor gene (hRyR2) underlie catecholaminergic polymorphic ventricular tachycardia. Circulation 2001 January 16;103(2):196-200.
Sumitomo N, Sakurada H, Taniguchi K, Matsumura M, Abe O, Miyashita M, Kanamaru H, Karasawa K, Ayusawa M, Fukamizu S, Nagaoka I, Horie M, Harada K, Hiraoka M. Association of atrial arrhythmia and sinus node dysfunction in patients with catecholaminergic polymorphic ventricular tachycardia. Circ J 2007 October;71(10):1606-9.
Sood S, Chelu MG, van Oort RJ, Skapura D, Santonastasi M, Dobrev D, Wehrens XH. Intracellular calcium leak due to FKBP12.6 deficiency in mice facilitates the inducibility of atrial fibrillation. Heart Rhythm 2008 July;5(7): 1047-54.
Postma AV, Denjoy I, Hoorntje TM, Lupoglazoff JM, Da CA, Sebillon P, Mannens MM, Wilde AA, Guicheney P. Absence of calsequestrin 2 causes severe forms of catecholaminergic polymorphic ventricular tachycardia. Circ Res 2002 October 18;91(8):e21-e26.
Iwasaki YK, Kato T, Xiong F, Shi YF, Naud P, Maguy A, Mizuno K, Tardif JC, Comtois P, Nattel S. Atrial fibrillation promotion with long-term repetitive obstructive sleep apnea in a rat model. J Am Coll Cardiol 2014 November 11;64(19):2013-23.
Guasch E, Benito B, Qi X, Cifelli C, Naud P, Shi Y, Mighiu A, Tardif JC, Tadevosyan A, Chen Y, Gillis MA, Iwasaki YK, Dobrev D, Mont L, Heximer S, Nattel S. Atrial fibrillation promotion by endurance exercise: demonstration and mechanistic exploration in an animal model. J Am Coll Cardiol 2013 July 2;62(1):68-77.
Savio-Galimberti E, Kannankeril PJ, Kor K, Blair M, Kupershmidt S, Darbar D. A novel mutation in human NPPA gene overexpressed in mice is associated with increased atrial fibrillation inducibility without atrial structural remodeling. Heart Rhythm. 11 (5S), -S6. 2014.
Faggioni M, Savio-Galimberti E, Venkataraman R, Hwang HS, Kannankeril PJ, Darbar D, Knollmann BC. Suppression of spontaneous Ca elevations prevents atrial fibrillation in calsequestrin 2-null hearts. Circ Arrhythm Electrophysiol 2014 April;7(2):313-20.
Davies SS, Talati M, Wang X, Mernaugh RL, Amamath V, Fessel J, Meyrick BO, Sheller J, Roberts LJ. Localization of isoketal adducts in vivo using a single-chain antibody. Free Radic Biol Med 2004 May 1;36(9):1163-74.
Greene MJ, Sam F, Soo Hoo PT, Patel RS, Seldin DC, Connors LH. Evidence for a functional role of the molecular chaperone clusterin in amyloidotic cardiomyopathy. Am J Pathol 2011 January;178(1):61-8.
Soderlund KA, Chivukula RR, Russell SD, Conte JV, Mudd JO, Halushka MK. Prognostic value of left ventricular apical tissue removed for HeartMate II left ventricular assist device placement. Cardiovasc Pathol 2009 July;18(4):217-22.
Wu J, Saleh MA, Kirabo A, Itani HA, Montaniel KR, Xiao L, Chen W, Memaugh RL, Cai H, Bernstein KE, Goronzy JJ, Weyand CM, Curci JA, Barbaro NR, Moreno H, Davies SS, Roberts LJ, Madhur MS, Harrison DG. Immune activation caused by vascular oxidation promotes fibrosis and hypertension. J Clin Invest 2016 April 1;126(4):1607.
Pucci A, Wharton J, Arbustini E, Grasso M, Diegoli M, Needleman P, Vigano M, Polak JM. Atrial amyloid deposits in the failing human heart display both atrial and brain natriuretic peptide-like immunoreactivity. J Pathol 1991 November;165(3):235-41.
Takemura G, Takatsu Y, Doyama K, Itoh H, Saito Y, Koshiji M, Ando F, Fujiwara T, Nakao K, Fujiwara H. Expression of atrial and brain natriuretic peptides and their genes in hearts of patients with cardiac amyloidosis. J Am Coll Cardiol 1998 March 15;31(4):754-65.
Iconomidou VA, Pheida D, Hamodraka ES, Antony C, Hoenger A, Hamodrakas SJ. An amyloidogenic determinant in N-terminal pro-brain natriuretic peptide (NT-proBNP): Implications for cardiac amyloidoses. Biopolymers 2012;98(1):67-75.
Louros NN, Iconomidou VA, Tsiolaki PL, Chrysina ED, Baltatzis GE, Patsouris ES, Hamodrakas SJ. An N-terminal pro-atrial natriuretic peptide (NT-proANP) 'aggregation-prone' segment involved in isolated atrial amyloidosis. FEBS Lett 2014 January 3;588(1):52-7.
Seldin DC, Skinner M. Arthritis accompanying systemic diseases. In: Harris ED, Budd RC, Genovese MC, Firestein GS, Sargent JS, Sledge CB, eds. Kelley's Textbook of Rheumatology. Seventh ed. Philadelphia: Elsevier Sanders; 2005. p. 1697-704.
Vesely DL. Atrial natriuretic peptide prohormone gene expression: hormones and diseases that upregulate its expression. IUBMB Life 2002 March;53(3):153-9.
Postma AV, van de Meerakker JB, Mathijssen IB, Barnett P, Christoffels VM, Ilgun A, Lam J, Wilde AA, Lekanne Deprez RH, Moorman AF. A gain-of-function TBX5 mutation is associated with atypical Holt-Oram syndrome and paroxysmal atrial fibrillation. Circ Res 2008 June 6;102(11):1433-42.
Hodgson-Zingman DM, Karst ML, Zingman LV, Heublein DM, Darbar D, Herron KJ, Ballew JD, de AM, Burnett JC, Jr., Olson TM. Atrial natriuretic peptide frameshift mutation in familial atrial fibrillation. N Engl J Med 2008 July 10;359(2):158-65.
Dickey DM, Yoder AR, Potter LR. A familial mutation renders atrial natriuretic Peptide resistant to proteolytic degradation. J Biol Chem 2009 July 17;284(29):19196-202.
Hua R, MacLeod SL, Polina I, Moghtadaei M, Jansen HJ, Bogachev O, O'Blenes SB, Sapp JL, Legare JF, Rose RA. Effects of wild-type and mutant forms of atrial natriuretic peptide on atrial electrophysiology and arrhythmogenesis. Circ Arrhythm Electrophysiol 2015 October;8(5):1240-54.
Prinsen JK, Savio-Galimberti E, Yermalitskaya LV, Sidorova TN, Barnett JV, Boutaud OG, Darbar D, Murray KT. A frame-shift mutation of NPPA promotes formation of cytotoxic preamyloid oligomers by mutant atrial natriuretic peptide. Heart Rhythm 12[5S], S4. 2015.
Christophersen IE, Ellinor PT. Genetics of atrial fibrillation: from families to genomes. J Hum Genet 2016 January;61(1):61-70.
Lashuel HA, Hartley D, Petre BM, Walz T, Lansbury PT, Jr. Neurodegenerative disease: amyloid pores from pathogenic mutations. Nature 2002 July 18;418(6895):291.
Janson J, Ashley RH, Harrison D, McIntyre S, Butler PC. The mechanism of islet amyloid polypeptide toxicity is membrane disruption by intermediate-sized toxic amyloid particles. Diabetes 1999 March;48(3):491-8.
Kagan BL. Membrane pores in the pathogenesis of neurodegenerative disease. Prog Mol Biol Transl Sci 2012;107:295-325.
Chrissobolis S, Faraci FM. Sex differences in protection against angiotensin II-induced endothelial dysfunction by manganese superoxide dismutase in the cerebral circulation. Hypertension 2010 April;55(4):905-10.
Sobocanec S, Balog T, Sverko V, Marotti T. Sex-dependent antioxidant enzyme activities and lipid peroxidation in ageing mouse brain. Free Radic Res 2003 July;37(7):743-8.
Westerheide SD, Morimoto RI. Heat shock response modulators as therapeutic tools for diseases of protein conformation. J Biol Chem 2005 September 30;280(39):33097-100.
Westerheide SD, Anckar J, Stevens SM, Jr., Sistonen L, Morimoto RI. Stress-inducible regulation of heat shock factor 1 by the deacetylase SIRT1. Science 2009 February 20;323(5917):1063-6.
Akerfelt M, Morimoto RI, Sistonen L. Heat shock factors: integrators of cell stress, development and lifespan. Nat Rev Mol Cell Biol 2010 August;11(8):545-55.
Hoogstra-Berends F, Meijering RA, Zhang D, Heeres A, Loen L, Seerden JP, Kuipers I, Kampinga HH, Henning RH, Brundel BJ. Heat shock protein-inducing compounds as therapeutics to restore proteostasis in atrial fibrillation. Trends Cardiovasc Med 2012 April;22(3):62-8.
Ooie T, Takahashi N, Saikawa T, Nawata T, Arikawa M, Yamanaka K, Hara M, Shimada T, Sakata T. Single oral dose of geranylgeranylacetone induces heat-shock protein 72 and renders protection against ischemia/reperfusion injury in rat heart. Circulation 2001 October 9;104(15):1837-43.
Brundel BJ, Ke L, Dijkhuis AJ, Qi X, Shiroshita-Takeshita A, Nattel S, Henning RH, Kampinga HH. Heat shock proteins as molecular targets for intervention in atrial fibrillation. Cardiovasc Res 2008 June 1;78(3):422-8.
Sakabe M, Shiroshita-Takeshita A, Maguy A, Brundel BJ, Fujiki A, Inoue H, Nattel S. Effects of a heat shock protein inducer on the atrial fibrillation substrate caused by acute atrial ischaemia. Cardiovasc Res 2008 April 1;78(1):63-70.
Brundel BJ, Henning RH, Ke L, Van G, I, Crijns HJ, Kampinga HH. Heat shock protein upregulation protects against pacing-induced myolysis in HL-1 atrial myocytes and in human atrial fibrillation. J Mol Cell Cardiol 2006 September;41(3):555-62.
Sanbe A, Daicho T, Mizutani R, Endo T, Miyauchi N, Yamauchi J, Tanonaka K, Glabe C, Tanoue A. Protective effect of geranylgeranylacetone via enhancement of HSPB8 induction in desmin-related cardiomyopathy. PLoS One 2009;4(4):e5351.
Mace LC, Yi Y, Hu X, Yang Z, Murray KT. Transcriptional profile of rapidly-stimulated atrial myocytes: Conservation with human atrial fibrillation. Circulation 114 (Suppl II), II-236. 2008.
Chopra N, Laver D, Davies SS, Knollmann BC. Amitriptyline activates cardiac ryanodine channels and causes spontaneous sarcoplasmic reticulum calcium release. Mol Pharmacol 2009 January;75(1):183-95.
Watanabe H, Chopra N, Laver D, Hwang HS, Davies SS, Roach DE, Duff HJ, Roden DM, Wilde AA, Knollmann BC. Flecainide prevents catecholaminergic polymorphic ventricular tachycardia in mice and humans. Nat Med 2009 April;15(4):380-3.
Kryshtal DO, Gryshchenko O, Gomez-Hurtado N, Knollmann BC. Impaired calcium-calmodulin-dependent inactivation of Cav1.2 contributes to loss of sarcoplasmic reticulum calcium release refractoriness in mice lacking calsequestrin 2. J Mol Cell Cardiol 2015 May;82:75-83.
Li N, Chiang DY, Wang S, Wang Q, Sun L, Voigt N, Respress JL, Ather S, Skapura DG, Jordan VK, Horrigan FT, Schmitz W, Muller FU, Valderrabano M, Nattel S, Dobrev D, Wehrens XH. Ryanodine receptor-mediated calcium leak drives progressive development of an atrial fibrillation substrate in a transgenic mouse model. Circulation 2014 March 25;129(12):1276-85.
Voigt N, Heijman J, Wang Q, Chiang DY, Li N, Karck M, Wehrens XH, Nattel S, Dobrev D. Cellular and molecular mechanisms of atrial arrhythmogenesis in patients with paroxysmal atrial fibrillation. Circulation 2014 January 14;129(2):145-56.
Shan J, Xie W, Betzenhauser M, Reiken S, Chen BX, Wronska A, Marks AR. Calcium leak through ryanodine receptors leads to atrial fibrillation in 3 mouse models of catecholaminergic polymorphic ventricular tachycardia. Circ Res 2012 August 31;111(6):708-17.
Yang Z, Shen W, Rottman JN, Wikswo JP, Murray KT. Rapid stimulation causes electrical remodeling in cultured atrial myocytes. J Mol Cell Cardiol 2005;38:299-308.
Yang Z, Murray KT. Ionic mechanisms of pacemaker activity in spontaneously contracting atrial HL-1 cells. J Cardiovasc Pharmacol 2011 January;57(1):28-36.
Pretorius M, Murray KT, Yu C, Byrne JG, Billings FT, Petracek MR, Greelish JP, Hoff SJ, Ball SK, Mishra V, Body SC, Brown NJ. Angiotensin-converting enzyme inhibition or mineralocorticoid receptor blockade do not affect prevalence of atrial fibrillation in patients undergoing cardiac surgery. Crit Care Med 2012 October;40(10):2805-12.

The invention thus being described, it would be obvious that the same can be varied in many ways. Such variations that would be obvious to one of ordinary skill in the art is to be considered as being part of this disclosure.

Unless otherwise indicated, all numbers expressing quantities of ingredients, properties such as reaction conditions, and so forth used in the Specification are to be understood as being modified in all instances by the term "about." Accordingly, unless indicated by the contrary, the numerical parameters set forth in the Specification and Claims are approximations that may vary depending upon the desired properties sought to be determined by the present invention.

## Claims

1. A pharmaceutical composition comprising a pharmaceutically acceptable carrier and a compound of the following formula, for use in the treatment, prevention, or amelioration of:
i) atrial fibrillation in a subject with or at risk of atrial fibrillation, thereby inhibiting or treating the atrial fibrillation; or
ii) atrial arrhythmias in a subject with or at risk of an atrial arrhythmia 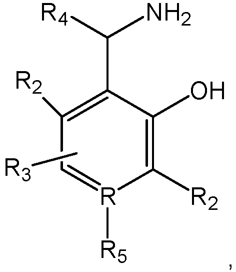 wherein:
R is C;
R₂ is independently H, hydroxy, halogen, nitro, CF₃, C₁₋₆ alkyl, C₁₋₆ alkoxy, C₃₋₁₀ cycloalkyl, C₃₋₈ membered ring containing C, O, S or N, optionally substituted with one or more R₂, R₃ and R_{4,} and may cyclize with one or more R_{2,} R₃, or R₅ to form an optionally substituted C₃₋₈ membered ring containing C, O, S or N;
R₃ is H, hydroxy, halogen, nitro, CF₃, C₁₋₆ alkyl, C₁₋₆ alkoxy, C₃₋₁₀ cycloalkyl, C₃₋₈ membered ring containing C, O, S or N, optionally substituted with one or more R₄, R₂ and R₃ may cyclize with one or more R₂ or R₅ to form an optionally substituted C₃₋₈ membered ring containing C, O, S or N;
R₄ is H, hydroxy, halogen, nitro, CF₃, C₁₋₆ alkyl, C₁₋₆ alkoxy, C₃₋₁₀ cycloalkyl, C₃₋₈ membered ring containing C, O, S or N, optionally substituted with one or more R₄, R₂ and R₃ may cyclize with one or more R_{2,} R_{3,} or R₅ to form an optionally substituted C₃₋₈ membered ring containing C, O, S or N;
R₅ is, H, hydroxy, halogen, nitro, CF₃, C₁₋₆ alkyl, C₁₋₆ alkoxy, C₃₋₁₀ cycloalkyl, C₃₋₈ membered ring containing C, O, S or N, optionally substituted with one or more R₄, R₂ and R₃ may cyclize with one or more R_{2,} R_{3,} or R₄ to form an optionally substituted C₃₋₈ membered ring containing C, O, S or N;
and stereoisomers thereof.

2. The composition for use according to claim 1, wherein the compound is of the following formula: 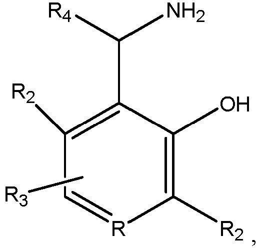 wherein:
R is CH;
R₂ is independently H, or substituted or unsubstituted C₁₋₆ alkyl;
R₃ is H, halogen, C₁₋₆ alkoxy, hydroxyl, or nitro;
R₄ is H, substituted or unsubstituted C₁₋₆ alkyl, or carboxyl; and pharmaceutically acceptable salts thereof.

3. The composition for use according to claim 1, wherein the compound is salicylamine (2-hydroxybenzylamine or 2-HOBA).

4. The composition for use according to claim 1, wherein the compound is 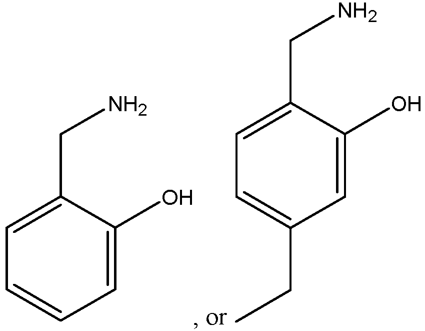 or a pharmaceutically acceptable salt thereof.

5. A compound of the following formula for use in treatment, prevention, or amelioration of atrial fibrillation in a subject with or at risk of atrial fibrillation, thereby inhibiting or treating the atrial fibrillation, the compound being co-administered to the subject with a drug having a known side effect of treating, preventing, or ameliorating atrial fibrillation 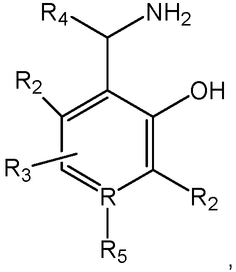 wherein:
R is C;
R₂ is independently H, hydroxy, halogen, nitro, CF₃, C₁₋₆ alkyl, C₁₋₆ alkoxy, C₃₋₁₀ cycloalkyl, C₃₋₈ membered ring containing C, O, S or N, optionally substituted with one or more R₂, R₃ and R_{4,} and may cyclize with one or more R_{2,} R_{3,} or R₅ to form an optionally substituted C₃₋₈ membered ring containing C, O, S or N;
R₃ is H, hydroxy, halogen, nitro, CF₃, C₁₋₆ alkyl, C₁₋₆ alkoxy, C₃₋₁₀ cycloalkyl, C₃₋₈ membered ring containing C, O, S or N, optionally substituted with one or more R₄, R₂ and R₃ may cyclize with one or more R₂ or R₅ to form an optionally substituted C₃₋₈ membered ring containing C, O, S or N;
R₄ is H, hydroxy, halogen, nitro, CF₃, C₁₋₆ alkyl, C₁₋₆ alkoxy, C₃₋₁₀ cycloalkyl, C₃₋₈ membered ring containing C, O, S or N, optionally substituted with one or more R₄, R₂ and R₃ may cyclize with one or more R_{2,} R_{3,} or R₅ to form an optionally substituted C₃₋₈ membered ring containing C, O, S or N;
R₅ is, H, hydroxy, halogen, nitro, CF₃, C₁₋₆ alkyl, C₁₋₆ alkoxy, C₃₋₁₀ cycloalkyl, C₃₋₈ membered ring containing C, O, S or N, optionally substituted with one or more R₄, R₂ and R₃ may cyclize with one or more R_{2,} R_{3,} or R₄ to form an optionally substituted C₃₋₈ membered ring containing C, O, S or N; and stereoisomers thereof.

6. The compound for use according to claim 5, wherein the compound is selected from the formula: 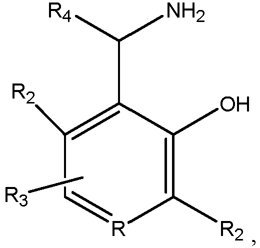 wherein:
R is CH;
R₂ is independently H, or substituted or unsubstituted C₁₋₆ alkyl;
R₃ is H, halogen, C₁₋₆ alkoxy, hydroxyl, or nitro;
R₄ is H, substituted or unsubstituted C₁₋₆ alkyl, or carboxyl; and pharmaceutically acceptable salts thereof.

7. The compound for use according to claim 5, wherein the compound is salicylamine (2-hydroxybenzylamine or 2-HOBA).

8. The compound for use according to claim 5, wherein the compound is selected from:
i) the formula: 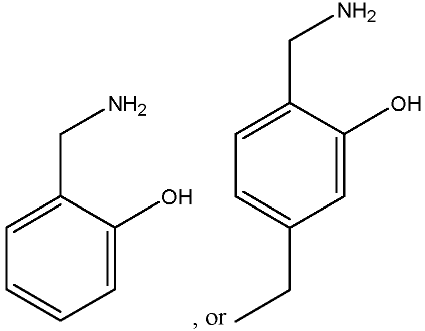 or a pharmaceutically acceptable salt thereof.

9. The composition for use according to claim 1, wherein the compound is:

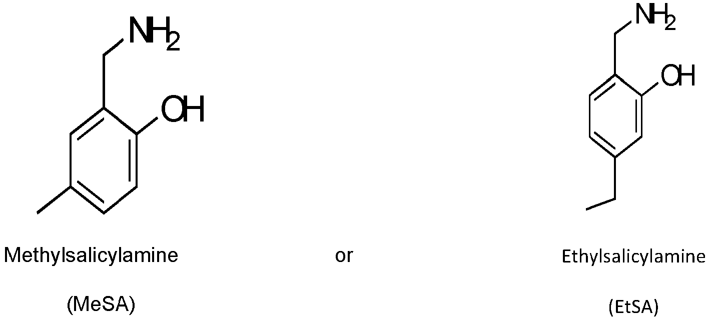

10. The compound for use according to claim 5, wherein the compound is:

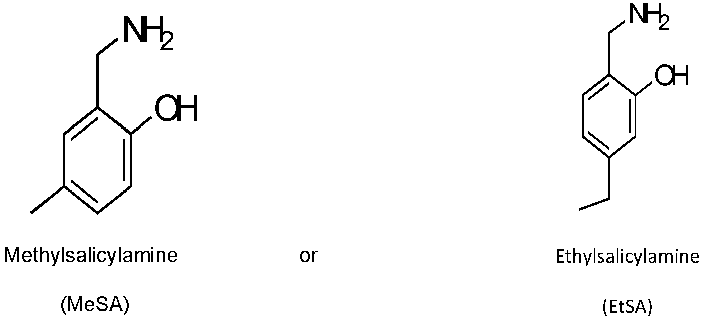

## Patentansprüche

1. Pharmazeutische Zusammensetzung, die einen pharmazeutisch annehmbaren Träger und eine Verbindung der folgenden Formel umfasst, zur Verwendung bei der Behandlung, Prävention oder Linderung von:
i) Vorhof-Flimmern in einem Individuum mit Vorhof-Flimmern oder einem Risiko dafür, wodurch das Vorhof-Flimmern gehemmt oder behandelt wird; oder
ii) Vorhof-Arrhythmien in einem Individuum mit einer Vorhof-Arrhythmie oder einem Risiko dafür, 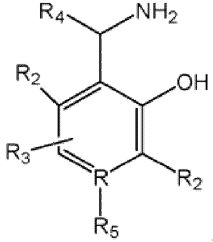 worin:
R C ist;
R₂ jeweils unabhängig H, Hydroxy, Halogen, Nitro, CF₃, C₁₋₆-Alkyl, C₁₋₆-Alkoxy, C₃₋₁₀-Cycloalkyl oder ein C₃₋₈-gliedriger Ring, der C, O, S oder N enthält, ist und gegebenenfalls mit einem oder mehreren R₂, R₃ und R₄ substituiert ist und mit einem oder mehreren R₂, R₃ oder R₅ zyklisieren kann, um einen gegebenenfalls substituierten C₃₋₈-gliedrigen Ring zu bilden, der C, O, S oder N enthält;
R₃ H, Hydroxy, Halogen, Nitro, CF₃, C₁₋₆-Alkyl, C₁₋₆-Alkoxy, C₃₋₁₀-Cycloalkyl oder ein C₃₋₈-gliedriger Ring, der C, O, S oder N enthält, ist und gegebenenfalls mit einem oder mehreren R₄, R₂ und R₃ substituiert ist und mit einem oder mehreren R₂ oder R₅ zyklisieren kann, um einen gegebenenfalls substituierten C₃₋₈-gliedrigen Ring zu bilden, der C, O, S oder N enthält;
R₄ H, Hydroxy, Halogen, Nitro, CF₃, C₁₋₆-Alkyl, C₁₋₆-Alkoxy, C₃₋₁₀-Cycloalkyl oder ein C₃₋₈-gliedriger Ring, der C, O, S oder N enthält, ist und gegebenenfalls mit einem oder mehreren R₄, R₂ und R₃ substituiert ist und mit einem oder mehreren R₂, R₃ oder R₅ zyklisieren kann, um einen gegebenenfalls substituierten C₃₋₈-gliedrigen Ring zu bilden, der C, O, S oder N enthält;
R₅ H, Hydroxy, Halogen, Nitro, CF₃, C₁₋₆-Alkyl, C₁₋₆-Alkoxy, C₃₋₁₀-Cycloalkyl oder ein C₃₋₈-gliedriger Ring, der C, O, S oder N enthält, ist und gegebenenfalls mit einem oder mehreren R₄, R₂ und R₃ substituiert ist und mit einem oder mehreren R₂, R₃ oder R₄ zyklisieren kann, um einen gegebenenfalls substituierten C₃₋₈-gliedrigen Ring zu bilden, der C, O, S oder N enthält;
und Stereoisomere davon.

2. Zusammensetzung zur Verwendung nach Anspruch 1, wobei die Verbindung die folgende Formel aufweist: 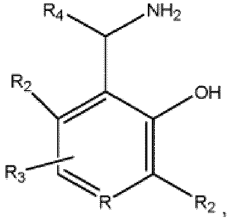 worin:
R CH ist;
R₂ jeweils unabhängig H oder substituiertes oder unsubstituiertes C₁₋₆-Alkyl ist;
R₃ H, Halogen, C₁₋₆-Alkoxy, Hydroxyl oder Nitro ist;
R₄ H, substituiertes oder unsubstituiertes C₁₋₆-Alkyl oder Carboxyl ist;
und pharmazeutisch annehmbare Salze davon.

3. Zusammensetzung zur Verwendung nach Anspruch 1, wobei die Verbindung Salicylamin (2-Hydroxybenzylamin oder 2-HOBA) ist.

4. Zusammensetzung zur Verwendung nach Anspruch 1, wobei die Verbindung die folgende: 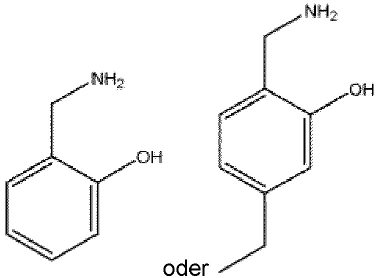 oder ein pharmazeutisch annehmbares Salz davon ist.

5. Verbindung der folgenden Formel zur Verwendung bei der Behandlung, Prävention oder Linderung von Vorhof-Flimmern in einem Individuum mit Vorhof-Flimmern oder mit einem Risiko dafür, wodurch das Vorhof-Flimmern inhibiert gehemmt oder behandelt wird, wobei die Verbindung zusammen mit einem Arzneimittel mit einer bekannten Nebenwirkung der Behandlung, Prävention oder Linderung von Vorhof-Flimmern an das Individuum verabreicht wird: 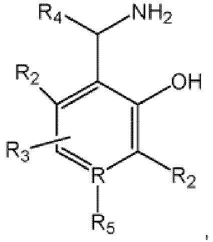 worin:
R C ist;
R₂ jeweils unabhängig H, Hydroxy, Halogen, Nitro, CF₃, C₁₋₆-Alkyl, C₁₋₆-Alkoxy, C₃₋₁₀-Cycloalkyl oder ein C₃₋₈-gliedriger Ring, der C, O, S oder N enthält, ist und gegebenenfalls mit einem oder mehreren R₂, R₃ und R₄ substituiert ist und mit einem oder mehreren R₂, R₃ oder R₅ zyklisieren kann, um einen gegebenenfalls substituierten C₃₋₈-gliedrigen Ring zu bilden, der C, O, S oder N enthält;
R₃ H, Hydroxy, Halogen, Nitro, CF₃, C₁₋₆-Alkyl, C₁₋₆-Alkoxy, C₃₋₁₀-Cycloalkyl oder ein C₃₋₈-gliedriger Ring, der C, O, S oder N enthält, ist und gegebenenfalls mit einem oder mehreren R₄, R₂ und R₃ substituiert ist und mit einem oder mehreren R₂ oder R₅ zyklisieren kann, um einen gegebenenfalls substituierten C₃₋₈-gliedrigen Ring zu bilden, der C, O, S oder N enthält;
R₄ H, Hydroxy, Halogen, Nitro, CF₃, C₁₋₆-Alkyl, C₁₋₆-Alkoxy, C₃₋₁₀-Cycloalkyl oder ein C₃₋₈-gliedriger Ring, der C, O, S oder N enthält, ist und gegebenenfalls mit einem oder mehreren R₄, R₂ und R₃ substituiert ist und mit einem oder mehreren R₂, R₃ oder R₅ zyklisieren kann, um einen gegebenenfalls substituierten C₃₋₈-gliedrigen Ring zu bilden, der C, O, S oder N enthält;
R₅ H, Hydroxy, Halogen, Nitro, CF₃, C₁₋₆-Alkyl, C₁₋₆-Alkoxy, C₃₋₁₀-Cycloalkyl oder ein C₃₋₈-gliedriger Ring, der C, O, S oder N enthält, ist und gegebenenfalls mit einem oder mehreren R₄, R₂ und R₃ substituiert ist und mit einem oder mehreren R₂, R₃ oder R₄ zyklisieren kann, um einen gegebenenfalls substituierten C₃₋₈-gliedrigen Ring zu bilden, der C, O, S oder N enthält;
und Stereoisomere davon.

6. Verbindung zur Verwendung nach Anspruch 5, wobei die Verbindung aus der folgenden Formel ausgewählt ist: 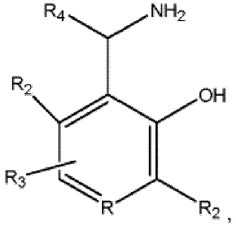 worin:
R CH ist;
R₂ jeweils unabhängig H oder substituiertes oder unsubstituiertes C₁₋₆-Alkyl ist;
R₃ H, Halogen, C₁₋₆-Alkoxy, Hydroxyl oder Nitro ist;
R₄ H, substituiertes oder unsubstituiertes C₁₋₆-Alkyl oder Carboxyl ist;
und pharmazeutisch annehmbare Salze davon.

7. Verbindung zur Verwendung nach Anspruch 5, wobei die Verbindung Salicylamin (2-Hydroxybenzylamin oder 2-HOBA) ist.

8. Verbindung zur Verwendung nach Anspruch 5, wobei die Verbindung aus:
i) der Formel: 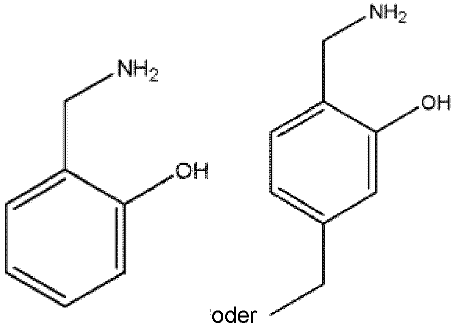 ausgewählt ist oder ein pharmazeutisch annehmbares Salz davon ist.

9. Zusammensetzung zur Verwendung nach Anspruch 1, wobei die Verbindung die folgende ist:

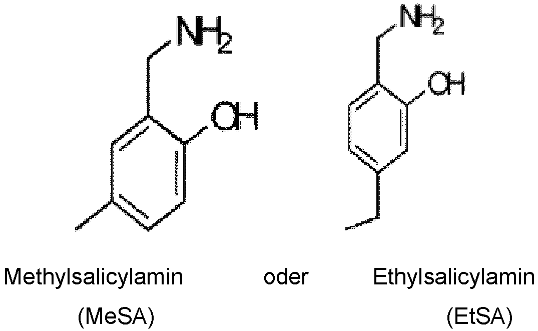

10. Verbindung zur Verwendung nach Anspruch 5, wobei die Verbindung die folgende ist:

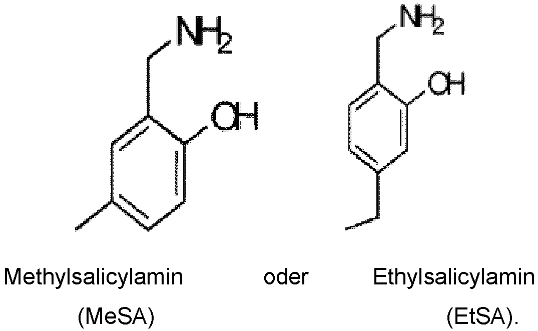

## Revendications

1. Composition pharmaceutique comprenant un support pharmaceutiquement acceptable et un composé de la formule suivante, pour utilisation dans le traitement, la prévention ou l'amélioration :
i) d'une fibrillation auriculaire chez un sujet souffrant, ou présentant le risque, d'une fibrillation auriculaire, inhibant ou traitant ainsi la fibrillation auriculaire ; ou
ii) d'arythmies auriculaires chez un sujet souffrant, ou présentant le risque, d'une arythmie auriculaire, 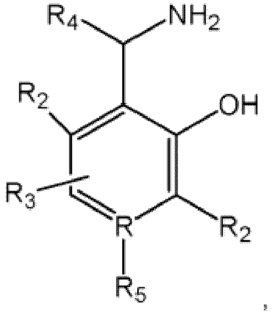 où :
R est C ;
R₂ est indépendamment H, hydroxy, halogène, nitro, CF₃, alkyle en C₁₋₆, alcoxy en C₁₋₆, cycloalkyle en C₃₋₁₀, cycle à chainons en C₃₋₈ contenant C, O, S ou N, facultativement substitué par un ou plusieurs R₂, R₃ et R₄, et peut se cycliser avec un ou plusieurs R₂, R₃ ou R₅ pour former un cycle à chainons en C₃₋₈ facultativement substitué contenant C, O, S ou N ;
R₃ est H, hydroxy, halogène, nitro, CF₃, alkyle en C₁₋₆, alcoxy en C₁₋₆, cycloalkyle en C₃₋₁₀, cycle à chainons en C₃₋₈ contenant C, O, S ou N, facultativement substitué par un ou plusieurs R₄, R₂ et R₃ peut se cycliser avec un ou plusieurs R₂ ou R₅ pour former un cycle à chainons en C₃₋₈ facultativement substitué contenant C, O, S ou N ;
R₄ est H, hydroxy, halogène, nitro, CF₃, alkyle en C₁₋₆, alcoxy en C₁₋₆, cycloalkyle en C₃₋₁₀, cycle à chainons en C₃₋₈ contenant C, O, S ou N, facultativement substitué par un ou plusieurs R₄, R₂ et R₃ peut se cycliser avec un ou plusieurs R₂, R₃ ou R₅ pour former un cycle à chainons en C₃₋₈ facultativement substitué contenant C, O, S ou N ;
R₅ est H, hydroxy, halogène, nitro, CF₃, alkyle en C₁₋₆, alcoxy en C₁₋₆, cycloalkyle en C₃₋₁₀, cycle à chainons en C₃₋₈ contenant C, O, S ou N, facultativement substitué par un ou plusieurs R₄, R₂ et R₃ peut se cycliser avec un ou plusieurs R₂, R₃ ou R₄ pour former un cycle à chainons en C₃₋₈ facultativement substitué contenant C, O, S ou N ;
et leurs stéréoisomères.

2. Composition pour utilisation selon la revendication 1, dans laquelle le composé est de la formule suivante : 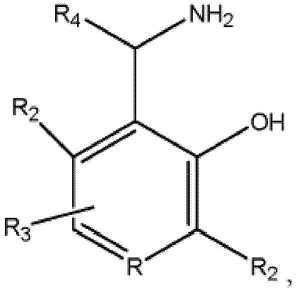 où :
R est CH ;
R₂ est indépendamment H, ou alkyle en C₁₋₆ substitué ou non substitué ;
R₃ est H, halogène, alcoxy en C₁₋₆, hydroxyle, ou nitro ;
R₄ est H, alkyle en C₁₋₆ substitué ou non substitué, ou carboxyle ; et leurs sels pharmaceutiquement acceptables.

3. Composition pour utilisation selon la revendication 1, dans laquelle le composé est une salicylamine (2-hydroxybenzylamine ou 2-HOBA).

4. Composition pour utilisation selon la revendication 1, dans laquelle le composé est 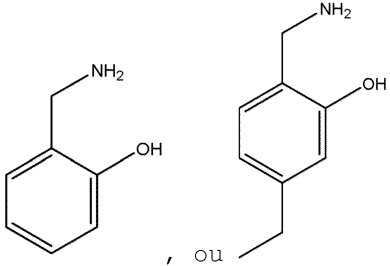 ou un sel pharmaceutiquement acceptable de celui-ci.

5. Composé de la formule suivante pour utilisation dans le traitement, la prévention ou l'amélioration d'une fibrillation auriculaire chez un sujet souffrant, ou présentant le risque, d'une fibrillation auriculaire, inhibant ou traitant ainsi la fibrillation auriculaire, le composé étant administré conjointement au sujet avec un médicament ayant un effet secondaire connu de traitement, prévention ou amélioration d'une fibrillation auriculaire 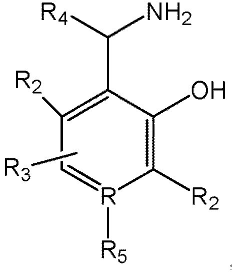 où :
R est C ;
R₂ est indépendamment H, hydroxy, halogène, nitro, CF₃, alkyle en C₁₋₆, alcoxy en C₁₋₆, cycloalkyle en C₃₋₁₀, cycle à chainons en C₃₋₈ contenant C, O, S ou N, facultativement substitué par un ou plusieurs R₂, R₃ et R₄, et peut se cycliser avec un ou plusieurs R₂, R₃ ou R₅ pour former un cycle à chainons en C₃₋₈ facultativement substitué contenant C, O, S ou N ;
R₃ est H, hydroxy, halogène, nitro, CF₃, alkyle en C₁₋₆, alcoxy en C₁₋₆, cycloalkyle en C₃₋₁₀, cycle à chainons en C₃₋₈ contenant C, O, S ou N, facultativement substitué par un ou plusieurs R₄, R₂ et R₃ peut se cycliser avec un ou plusieurs R₂ ou R₅ pour former un cycle à chainons en C₃₋₈ facultativement substitué contenant C, O, S ou N ;
R₄ est H, hydroxy, halogène, nitro, CF₃, alkyle en C₁₋₆, alcoxy en C₁₋₆, cycloalkyle en C₃₋₁₀, cycle à chainons en C₃₋₈ contenant C, O, S ou N, facultativement substitué par un ou plusieurs R₄, R₂ et R₃ peut se cycliser avec un ou plusieurs R₂, R₃ ou R₅ pour former un cycle à chainons en C₃₋₈ facultativement substitué contenant C, O, S ou N ;
R₅ est H, hydroxy, halogène, nitro, CF₃, alkyle en C₁₋₆, alcoxy en C₁₋₆, cycloalkyle en C₃₋₁₀, cycle à chainons en C₃₋₈ contenant C, O, S ou N, facultativement substitué par un ou plusieurs R₄, R₂ et R₃ peut se cycliser avec un ou plusieurs R₂, R₃ ou R₄ pour former un cycle à chainons en C₃₋₈ facultativement substitué contenant C, O, S ou N ; et leurs stéréoisomères.

6. Composé pour utilisation selon la revendication 5, dans lequel le composé est sélectionné parmi la formule : 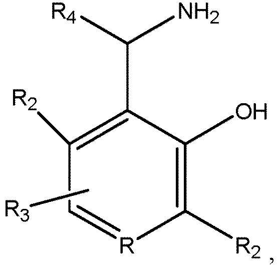 où :
R est CH ;
R₂ est indépendamment H, ou alkyle en C₁₋₆ substitué ou non substitué ;
R₃ est H, halogène, alcoxy en C₁₋₆, hydroxyle, ou nitro ;
R₄ est H, alkyle en C₁₋₆ substitué ou non substitué, ou carboxyle ; et leurs sels pharmaceutiquement acceptables.

7. Composé pour utilisation selon la revendication 5, dans lequel le composé est une salicylamine (2-hydroxybenzylamine ou 2-HOBA).

8. Composé pour utilisation selon la revendication 5, dans lequel le composé est sélectionné parmi :
i) la formule : 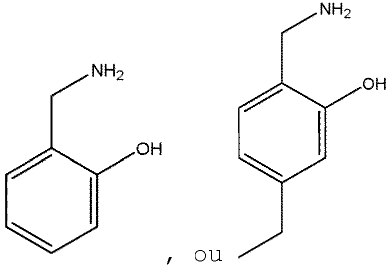 ou un sel pharmaceutiquement acceptable de celui-ci.

9. Composition pour utilisation selon la revendication 1, dans laquelle le composé est :

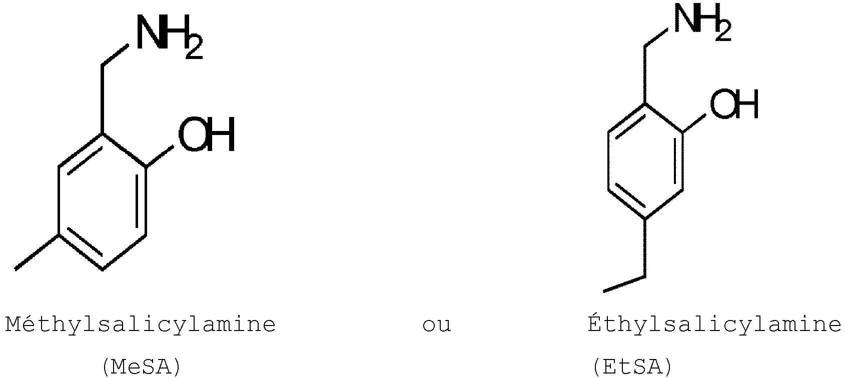

10. Composé pour utilisation selon la revendication 5, dans lequel le composé est :
